# EUROPEAN PATENT APPLICATION

(11) **EP 1 816 123 A1**
(43) Date of publication of application: **08.08.2007**
(21) Application number: 07010143.1
(22) Date of filing: 12.09.2003
(51) Int. Cl.: C07D 211/46, C07D 401/06, C07D 401/14

(54) **Intermediates for the production of pharmaceutically active compounds**

(30) Priority: 24.09.2002 SE 0202838
(62) Divisional of application: 03798620.5
(71) Applicant: AstraZeneca AB, 151 85 Södertälje (SE)
(72) Inventor: Luckhurst, Christopher, Loughborough Leicestershire LE11 5RH (GB); Perry, Matthew, Loughborough Leicestershire LE11 5RH (GB); Springthorpe, Brian, Loughborough Leicestershire LE11 5RH (GB)

(57) **Abstract**

The present invention provides a compound of a formula (A), (B) or (C): 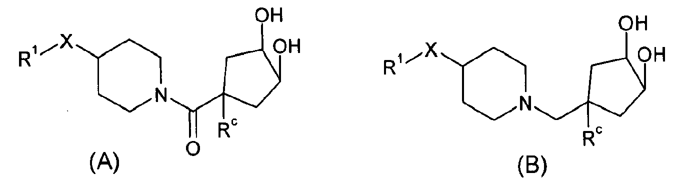 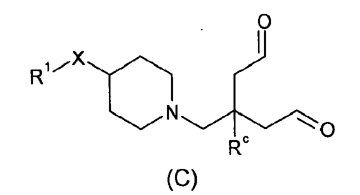 which is useful for making a compound of formula (I): 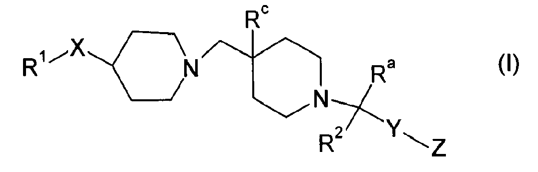 wherein Z is CO₂R³. The compounds of formula (I) are useful in the treatment of a chemokine (such as CCR3) or H1 mediated disease state.

## Description

The present invention concerns piperidine derivatives having pharmaceutical activity, to processes for preparing such derivatives, to pharmaceutical compositions comprising such derivatives and to the use of such derivatives as active therapeutic agents.

Pharmaceutically active piperidine derivatives are disclosed in WO99/38514, WO99/04794 and WO00/35877.

Histamine is a basic amine, 2-(4-imidazolyl)-ethylamine, and is formed from histidine by histidine decarboxylase. It is found in most tissues of the body, but is present in high concentrations in the lung, skin and in the gastrointestinal tract. At the cellular level inflammatory cells such as mast cells and basophils store large amounts of histamine. It is recognised that the degranulation of mast cells and basophils and the subsequent release of histamine is a fundamental mechanism responsible for the clinical manifestation of an allergic process. Histamine produces its actions by an effect on specific histamine G-protein coupled receptors, which are of three main types, H1, H2 and H3. Histamine H1 antagonists comprise the largest class of medications used in the treatment of patients with allergic disorders, such as rhinitis and urticaria. H1 antagonists are useful in controlling the allergic response by for example blocking the action of histamine on post-capillary venule smooth muscle, resulting in decreased vascular permeability, exudation and oedema. The antagonists also produce blockade of the actions of histamine on the H1 receptors on c-type nociceptive nerve fibres, resulting in decreased itching and sneezing.

Viral infections are known to cause lung inflammation. It has been shown experimentally that the common cold increases mucosal output of eotaxin in the airways. Instillation of eotaxin into the nose can mimic some of the signs and symptoms of a common cold. (See, Greiff L et al Allergy (1999) 54(11) 1204-8 [Experimental common cold increase mucosal output of eotaxin in atopic individuals] and Kawaguchi M et al Int. Arch. Allergy Immunol. (2000) 122 S144 [Expression of eotaxin by normal airway epithelial cells after virus A infection].)

Chemokines are chemotactic cytokines that are released by a wide variety of cells to attract macrophages, T cells, eosinophils, basophils and neutrophils to sites of inflammation and also play a rôle in the maturation of cells of the immune system. Chemokines play an important rôle in immune and inflammatory responses in various diseases and disorders, including asthma and allergic diseases, as well as autoimmune pathologies such as rheumatoid arthritis and atherosclerosis. These small secreted molecules are a growing superfamily of 8-14 kDa proteins characterised by a conserved four cysteine motif. The chemokine superfamily can be divided into two main groups exhibiting characteristic structural motifs, the Cys-X-Cys (C-X-C, or α) and Cys-Cys (C-C, or β) families. These are distinguished on the basis of a single amino acid insertion between the NH-proximal pair of cysteine residues and sequence similarity.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils such as human monocyte chemotactic proteins 1-3 (MCP-1, MCP-2 and MCP-3), RANTES (Regulated on Activation, Normal T Expressed and Secreted), eotaxin and the macrophage inflammatory proteins 1α and 1β (MIP-1α and MIP-1β).

Studies have demonstrated that the actions of the chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CCR10, CXCR1, CXCR2, CXCR3 and CXCR4. These receptors represent good targets for drug development since agents which modulate these receptors would be useful in the treatment of disorders and diseases such as those mentioned above.

The present invention provides a compound of formula (I): 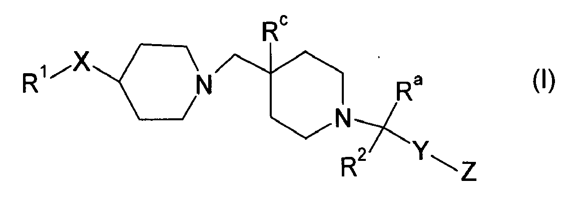 wherein:
X is CH₂, C(O), O, S, S(O), S(O)₂ or NR³;
Y is a bond, C₁₋₆ alkylene (optionally substituted by C₁₋₄ alkyl or phenyl), phenylene (optionally substituted by halogen, hydroxy, C₁₋₄ alkyl or C₁₋₄ alkoxy) or heterocyclylene (optionally substituted by halogen, hydroxy, C₁₋₄ alkyl or C₁₋₄ alkoxy);
Z is CO₂R^{b}, NHS(O)₂CF₃, S(O)₂OH, OCH₂CO₂R^{b} or tetrazolyl;
R¹ is hydrogen, C₁₋₆ alkyl, aryl or heterocyclyl;
R² is hydrogen, C₁₋₆ alkyl, aryl or heterocyclyl;
R^{a} and R^{b} are, independently, hydrogen or C₁₋₄ alkyl; or when R² is aryl or heterocyclyl R^{a} may be C₂₋₃ alkylene forming a ring with an ortho position on R²;
R^{c} is hydrogen or hydroxy;
wherein, unless stated otherwise, the foregoing aryl and heterocyclyl moieties are optionally substituted by: halogen, cyano, nitro, hydroxy, oxo, S(O)pR⁴, OC(O)NR⁵R⁶, NR⁷R⁸, NR⁹C(O)R¹⁰, NR¹¹C(O)NR¹²R¹³, S(O)₂NR¹⁴R¹⁵, NR¹⁶S(O)₂R¹⁷, C(O)NR¹⁸R¹⁹, C(O)R²⁰, CO₂R²¹, NR²²CO₂R²³, C₁₋₆ alkyl, CF₃, C₁₋₆ alkoxy(C₁₋₆)alkyl, C₁₋₆ alkoxy, OCF₃, C₁₋₆ alkoxy(C₁₋₆)alkoxy, C₁₋₆ alkylthio, C₂₋₆ alkenyl, C₂₋₆ alkynyl, C₃₋₁₀ cycloalkyl (itself optionally substituted by C₁₋₄ alkyl or oxo), methylenedioxy, difluoromethylenedioxy, phenyl, phenyl(C₁₋₄)alkyl, phenoxy, phenylthio, phenyl(C₁₋₄)alkoxy, heterocyclyl, heterocyclyl(C₁₋₄)alkyl, heterocyclyloxy or heterocyclyl(C₁₋₄)alkoxy; wherein any of the immediately foregoing phenyl and heterocyclyl moieties are optionally substituted with halogen, hydroxy, nitro, S(O)_{q}(C₁₋₄ alkyl), S(O)₂NH₂, S(O)₂NH(C₁₋₄ alkyl), S(O)₂N(C₁₋₄ alkyl)₂ (and these alkyl groups may join to form a ring as described for R⁵ and R⁶ below), cyano, C₁₋₄ alkyl, C₁₋₄ alkoxy, C(O)NH₂, C(O)NH(C₁₋₄ alkyl), C(O)N(C₁₋₄ alkyl)₂(and these alkyl groups may join to form a ring as described for R⁵ and R⁶ below), CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃; p and q are, independently, 0, 1 or 2;
R³, R⁵, R⁶, R⁷, R⁸, R⁹, R¹⁰, R¹¹, R¹², R¹³, R¹⁴, R¹⁵, R¹⁶, R¹⁸, R¹⁹, R²⁰, R²¹ and R²² are, independently, hydrogen, C₁₋₆ alkyl (optionally substituted by halogen, hydroxy or C₃₋₁₀ cycloalkyl), CH₂(C₂₋₆ alkenyl), phenyl (itself optionally substituted by halogen, hydroxy, nitro, NH₂, NH(C₁₋₄ alkyl), N(C₁₋₄ alkyl)₂ (and these alkyl groups may join to form a ring as described for R⁵ and R⁶ below), S(O)₂(C₁₋₄ alkyl), S(O)₂NH₂, S(O)₂NH(C₁₋₄ alkyl), S(O)₂N(C₁₋₄ alkyl)₂ (and these alkyl groups may join to form a ring as described for R⁵ and R⁶ below), cyano, C₁₋₄ alkyl, C₁₋₄ alkoxy, C(O)NH₂, C(O)NH(C₁₋₄ alkyl), C(O)N(C₁₋₄ alkyl)₂ (and these alkyl groups may join to form a ring as described for R⁵ and R⁶ below), CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃) or heterocyclyl (itself optionally substituted by halogen, hydroxy, nitro, NH₂, NH(C₁₋₄ alkyl), N(C₁₋₄ alkyl)₂ (and these alkyl groups may join to form a ring as described for R⁵ and R⁶ below), S(O)₂(C₁₋₄ alkyl), S(O)₂NH₂, S(O)₂NH(C₁₋₄ alkyl), S(O)₂N(C₁₋₄ alkyl)₂ (and these alkyl groups may join to form a ring as described for R⁵ and R⁶ below), cyano, C₁₋₄ alkyl, C₁₋₄ alkoxy, C(O)NH₂, C(O)NH(C₁₋₄ alkyl), C(O)N(C₁₋₄ alkyl)₂(and these alkyl groups may join to form a ring as described for R⁵ and R⁶ below), CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃);
alternatively NR⁵R⁶, NR⁷R⁸, NR¹²R¹³, NR¹⁴R¹⁵, NR¹⁸R¹⁹, may, independently, form a 4-7 membered heterocyclic ring, azetidine, pyrrolidine, piperidine, azepine, morpholine or piperazine, the latter optionally substituted by C₁₋₄ alkyl on the distal nitrogen;
R⁴, R¹⁷ and R²³ are, independently, C₁₋₆ alkyl (optionally substituted by halogen, hydroxy or C₃₋₁₀ cycloalkyl), CH₂(C₂₋₆ alkenyl), phenyl (itself optionally substituted by halogen, hydroxy, nitro, NH₂, NH(C₁₋₄ alkyl), N(C₁₋₄ alkyl)₂ (and these alkyl groups may join to form a ring as described for R⁵ and R⁶ above), S(O)₂(C₁₋₄ alkyl), S(O)₂NH₂, S(O)₂NH(C₁₋₄ alkyl), S(O)₂N(C₁₋₄ alkyl)₂ (and these alkyl groups may join to form a ring as described for R⁵ and R⁶ above), cyano, C₁₋₄ alkyl, C₁₋₄ alkoxy, C(O)NH₂, C(O)NH(C₁₋₄ alkyl), C(O)N(C₁₋₄ alkyl)₂ (and these alkyl groups may join to form a ring as described for R⁵ and R⁶ above), CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃) or heterocyclyl (itself optionally substituted by halogen, hydroxy, nitro, NH₂, NH(C₁₋₄ alkyl), N(C₁₋₄ alkyl)₂ (and these alkyl groups may join to form a ring as described for R⁵ and R⁶ above), S(O)₂(C₁₋₄ alkyl), S(O)₂NH₂, S(O)₂NH(C₁₋₄ alkyl), S(O)₂N(C₁₋₄ alkyl)₂ (and these alkyl groups may join to form a ring as described for R⁵ and R⁶ above), cyano, C₁₋₄ alkyl, C₁₋₄ alkoxy, C(O)NH₂, C(O)NH(C₁₋₄ alkyl), C(O)N(C₁₋₄ alkyl)₂ (and these alkyl groups may join to form a ring as described for R⁵ and R⁶ above), CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃);
or an N-oxide thereof; or a pharmaceutically acceptable salt thereof; or a solvate thereof.

Certain compounds of the present invention can exist in different isomeric forms (such as enantiomers, diastereomers, geometric isomers or tautomers). The present invention covers all such isomers and mixtures thereof in all proportions.

Suitable salts include acid addition salts such as a hydrochloride, dihydrochloride, hydrobromide, sulfate, phosphate, acetate, diacetate, fumarate, maleate, tartrate, citrate, oxalate, methanesulfonate, ethanesulfonate or *p*-toluenesulfonate. Salts also include metal salts, such as an alkali metal salt (for example a sodium or potassium salt) or an alkaline earth metal salt (for example magnesium or calcium).

The compounds of the invention may exist as solvates (such as hydrates) and the present invention covers all such solvates.

Halogen includes fluorine, chlorine, bromine and iodine. Halogen is, for example, fluorine or chlorine.

Alkyl groups and moieties are straight or branched chain and comprise, for example, 1 to 6 (such as 1 to 4) carbon atoms. Examples of alkyl groups are methyl, ethyl, n-propyl, iso-propyl or tert-butyl.

Alkylene is a straight carbon chain of 1 to 6 carbons, which is optionally substituted. Alkylene includes CH₂ or CH₂CH₂, and when substituted by alkyl (for example) it can be CH(CH₃) or CH₂C(CH₃)₂.

Alkenyl groups comprise, for example, 2 to 6 (such as 2 to 4) carbon atoms. Examples of alkenyl groups are vinyl or allyl.

Alkynyl groups comprise, for example, 2 to 6 (such as 2 to 4) carbon atoms. An example of an alkynyl group is propargyl.

In one embobiment cycloalkyl groups comprise from 3 to 10 (such as 3 to 8, for example 3 to 6) carbon atoms and are mono-, bi or tricyclic. Cycloalkyl is, for example, cyclopropyl, cyclopentyl, cyclohexyl, norbornyl or camphoryl. The cycloalkyl ring is optionally fused to a benzene ring (for example forming a bicyclo[4.2.0]octa-1,3,5-trienyl or indanyl ring system).

In another embodiment cycloalkenyl comprises from 3 to 8 (such as from 3 to 6) carbon atoms and is, for example, monocyclic. Cycloalkenyl is, for example, cyclopentenyl or cyclohexenyl.

Aryl includes phenyl or naphthyl.

Heterocyclyl is an aromatic or non-aromatic 5 or 6 membered ring, optionally fused to one or more other rings, comprising at least one heteroatom selected from the group comprising nitrogen, oxygen and sulfur; or an N-oxide thereof, or an S-oxide or S-dioxide thereof. Heterocyclyl is, for example, furyl, thienyl (also known as thiophenyl), pyrrolyl, 2,5-dihydropyrrolyl, thiazolyl, pyrazolyl, oxazolyl, isoxazolyl, imidazolyl, piperidinyl, morpholinyl, pyridinyl, dihydropyridinyl (for example in a 6-oxo-1,6-dihydro-pyridinyl moiety), pyrimidinyl, indolyl, 2,3-dihydroindolyl, benzo[b]furyl (also known as benzfuryl), benz[b]thienyl (also known as benzthienyl or benzthiophenyl), 2,3-dihydrobenz[b]thienyl (for example in a 1-dioxo-2,3-dihydrobenz[b]thienyl moiety), indazolyl, benzimidazolyl, benztriazolyl, benzoxazolyl, benzthiazolyl (for example in a 1H-benzthiazol-2-one-yl moiety), 2,3-dihydrobenzthiazolyl (for example in a 2,3-dihydrobenzthiazol-2-one-yl moiety), 1,2,3-benzothiadiazolyl, an imidazopyridinyl (such as imidazo[1,2a]pyridinyl), thieno[3,2-b]pyridin-6-yl 1,2,3-benzoxadiazolyl (also known as benzo[1,2,3]thiadiazolyl), 2,1,3-benzothiadiazolyl, benzofurazan (also known as 2,1,3-benzoxadiazolyl), quinoxalinyl, dihydro-1-benzopyryliumyl (for example in a coumarinyl or a chromonyl moiety), 3,4-dihydro-1H-2,1-benzothiazinyl (for example in a 2-dioxo-3,4-dihydro-1H-2,1-benzothiazinyl moiety), a pyrazolopyridine (for example 1H-pyrazolo[3,4-b]pyridinyl), a purine (for example in a 3,7-dihydro-purin-2,6-dione-8-yl moiety), quinolinyl, isoquinolinyl, dihydroisoquinolinyl (for example in a 2H-isoquinolin-1-one-yl moiety), a naphthyridinyl (for example [1,6]naphthyridinyl or [1,8]naphthyridinyl), a dihydro[1,8]naphthyridinyl (for example in a 1H-[1,8]naphthyridin-4-one-yl moiety), a benzothiazinyl, a dihydrobenzothiazinyl (for example in a 4H-benzo[1,4]thiazin-3-one-yl moiety), benzo[d]imidazo[2,1-b]thiazol-2-yl or dibenzothiophenyl (also known as dibenzothienyl); or an N-oxide thereof, or an S-oxide or S-dioxide thereof.

An N-oxide of a compound of formula (I) is, for example, a 1-oxy-[1,4']bipiperidinyl-1'-yl compound.

Phenylene is a phenyl ring joining the carbon to which, *inter alia,* R² is attached, and the group Z (such as in Example 42 below).

Heterocyclylene is a heterocyclyl ring joining the carbon to which, *inter alia,* R² is attached, and the group Z (such as in Example 48 below). Heterocyclylene is, for example, pyridyl or oxazolyl.

When R² is aryl or heterocyclyl and R^{a} is C₂₋₃ alkylene which forms a ring with an ortho position on R² the resulting compound comprises, for example, an indene ring system. (See, for example, Example 41.)

Phenyl(C₁₋₄ alkyl) is, for example, benzyl or 2-phenyleth-1-yl.

Phenyl(C₁₋₄ alkoxy) is, for example, benzyloxy or 2-phenyleth-1-yloxy.

Heterocyclyl(C₁₋₄ alkyl) is, for example, pyridylmethyl or 2-pyridyleth-1-yl.

Heterocyclyl(C₁₋₄ alkoxy) is, for example, pyridyloxy or 2-pyridyleth-1-yloxy.

In one particular aspect the invention provides a compound of formula (Ia): 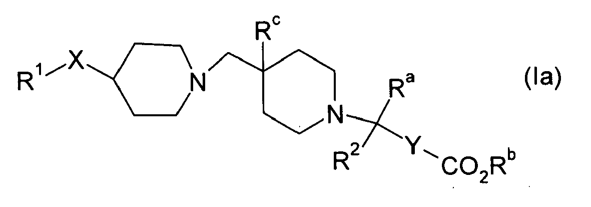 wherein:
X is CH₂, C(O), O, S, S(O), S(O)₂ or NR³;
Y is a bond, C₁₋₆ alkylene (optionally substituted by C₁₋₄ alkyl or phenyl) or phenylene (optionally substituted by halogen, hydroxy, C₁₋₄ alkyl or C₁₋₄ alkoxy);
R^{a} and R^{b} are, independently, hydrogen or C₁₋₄ alkyl;
R^{c} is hydrogen or hydroxy;
R¹ is hydrogen, C₁₋₆ alkyl, aryl or heterocyclyl;
R² is hydrogen, C₁₋₆ alkyl, aryl or heterocyclyl;
wherein, unless stated otherwise, the foregoing aryl and heterocyclyl moieties are optionally substituted by: halogen, cyano, nitro, hydroxy, oxo, S(O)ₚR⁴, OC(O)NR⁵R⁶, NR⁷R⁸, NR⁹C(O)R¹⁰, NR¹¹C(O)NR¹²R¹³, S(O)₂NR¹⁴R¹⁵, NR¹⁶S(O)₂R¹⁷, C(O)NR¹⁸R¹⁹, C(O)R²⁰, CO₂R²¹, NR²²CO₂R²³, C₁₋₆ alkyl, CF₃, C₁₋₆ alkoxy(C₁₋₆)alkyl, C₁₋₆ alkoxy, OCF₃, C₁₋₆ alkoxy(C₁₋₆)alkoxy, C₁₋₆ alkylthio, C₂₋₆ alkenyl, C₂₋₆ alkynyl, C₃₋₁₀ cycloalkyl (itself optionally substituted by C₁₋₄ alkyl or oxo), methylenedioxy, difluoromethylenedioxy, phenyl, phenyl(C₁₋₄)alkyl, phenoxy, phenylthio, phenyl(C₁₋₄)alkoxy, heterocyclyl, heterocyclyl(C₁₋₄)alkyl, heterocyclyloxy or heterocyclyl(C₁₋₄)alkoxy; wherein any of the immediately foregoing phenyl and heterocyclyl moieties are optionally substituted with halogen, hydroxy, nitro, S(O)_{q}(C₁₋₄ alkyl), S(O)₂NH₂, S(O)₂NH(C₁₋₄ alkyl), S(O)₂N(C₁₋₄ alkyl)₂ (and these alkyl groups may join to form a ring as described for R⁵ and R⁶ below), cyano, C₁₋₄ alkyl, C₁₋₄ alkoxy, C(O)NH₂, C(O)NH(C₁₋₄ alkyl), C(O)N(C₁₋₄ alkyl)₂(and these alkyl groups may join to form a ring as described for R⁵ and R⁶ below), CO₂H, CO₂(C₁₋₄ alkyl), NHC(O)(C₁₋₄ alkyl), NHS(O)₂(C₁₋₄ alkyl), C(O)(C)₁₋₄ alkyl), CF₃ or OCF₃; p and q are, independently, 0, 1 or 2;
R³, R⁵, R⁶, R⁷, R⁸, R⁹, R¹⁰, R¹¹, R¹², R¹³, R¹⁴, R¹⁵, R¹⁶, R¹⁸, R¹⁹, R²⁰, R²¹ and R²² are, independently, hydrogen, C₁₋₆ alkyl (optionally substituted by halogen, hydroxy or C₃₋₁₀ cycloalkyl), CH₂(C₂₋₆ alkenyl), phenyl (itself optionally substituted by halogen, hydroxy, nitro, NH₂, NH(C₁₋₄ alkyl), N(C₁₋₄ alkyl)₂, S(O)₂(C₁₋₄ alkyl), S(O)₂NH₂, S(O)₂NH(C₁₋₄ alkyl), S(O)₂N(C₁₋₄ alkyl)₂ (and these alkyl groups may join to form a ring as described for R⁵ and R⁶ below), cyano, C₁₋₄ alkyl, C₁₋₄ alkoxy, C(O)NH₂, C(O)NH(C₁₋₄ alkyl), C(O)N(C₁₋₄ alkyl)₂ (and these alkyl groups may join to form a ring as described for R⁵ and R⁶ below), CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃) or heterocyclyl (itself optionally substituted by halogen, hydroxy, nitro, NH₂, NH(C₁₋₄ alkyl), N(C₁₋₄ alkyl)₂, S(O)₂(C₁₋₄ alkyl), S(O)₂NH₂, S(O)₂NH(C₁₋₄ alkyl), S(O)₂N(C₁₋₄ alkyl)₂ (and these alkyl groups may join to form a ring as described for R⁵ and R⁶ below), cyano, C₁₋₄ alkyl, C₁₋₄ alkoxy, C(O)NH₂, C(O)NH(C₁₋₄ alkyl), C(O)N(C₁₋₄ alkyl)₂ (and these alkyl groups may join to form a ring as described for R⁵ and R⁶ below), CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃);
alternatively NR⁵R⁶, NR⁷R⁸, NR¹²R¹³, NR¹⁴R¹⁵, NR¹⁸R¹⁹, may, independently, form a 4-7 membered heterocyclic ring, azetidine, pyrrolidine, piperidine, azepine, morpholine or piperazine, the latter optionally substituted by C₁₋₄ alkyl on the distal nitrogen;
R⁴, R¹⁷ and R²³ are, independently, C₁₋₆ alkyl (optionally substituted by halogen, hydroxy or C₃₋₁₀ cycloalkyl), CH₂(C₂₋₆ alkenyl), phenyl (itself optionally substituted by halogen, hydroxy, nitro, NH₂, NH(C₁₋₄ alkyl), N(C₁₋₄ alkyl)₂ (and these alkyl groups may join to form a ring as described for R⁵ and R⁶ above), S(O)₂(C₁₋₄ alkyl), S(O)₂NH₂, S(O)₂NH(C₁₋₄ alkyl), S(O)₂N(C₁₋₄ alkyl)₂ (and these alkyl groups may join to form a ring as described for R⁵ and R⁶ above), cyano, C₁₋₄ alkyl, C₁₋₄ alkoxy, C(O)NH₂, C(O)NH(C₁₋₄ alkyl), C(O)N(C₁₋₄ alkyl)₂ (and these alkyl groups may join to form a ring as described for R⁵ and R⁶ above), CO₂H, CO₂(C₁₋₄ alkyl), NHC(O)(C₁₋₄ alkyl), NES(O)₂(C₁₋₄ alkyl), C(O)(C₁₋₄ alkyl), CF₃ or OCF₃) or heterocyclyl (itself optionally substituted by halogen, hydroxy, nitro, NH₂, NH(C₁₋₄ alkyl), N(C₁₋₄ alkyl)₂ (and these alkyl groups may join to form a ring as described for R⁵ and R⁶ above), S(O)₂(C₁₋₄ alkyl), S(O)₂NH₂, S(O)₂NH(C₁₋₄ alkyl), S(O)₂N(C₁₋₄ alkyl)₂ (and these alkyl groups may join to form a ring as described for R⁵ and R⁶ above), cyano, C₁₋₄ alkyl, C₁₋₄ alkoxy, C(O)NH₂, C(O)NH(C₁₋₄ alkyl), C(O)N(C₁₋₄ alkyl)₂ (and these alkyl groups may join to form a ring as described for R⁵ and R⁶ above), CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃);
or an N-oxide thereof; or a pharmaceutically acceptable salt thereof; or a solvate thereof.

In another aspect the invention provides a compound wherein X is O.

In yet another aspect R¹ is phenyl optionally substituted (for example independently mono- or di-substituted) with halogen (for example chlorine or fluorine), C₁₋₄ alkyl (for example methyl) or C₁₋₄ alkoxy (for example methoxy).

In a further aspect R¹ is phenyl optionally substituted (for example with one, two or three of the same or different) with fluorine, chlorine, C₁₋₄ alkyl (for example methyl) or C₁₋₄ alkoxy (for example methoxy). In a still further aspect R¹ is phenyl substituted by one, two or three (for example two or three) substituents independently selected from: fluorine, chlorine and methyl. For example R¹ is 3,4-dichlorophenyl, 2,4-dichloro-3-methylphenyl, 3,4-dichloro-2-methylphenyl, 2,4-dichlorophenyl, 4-chloro-2-methylphenyl or 2-chloro-4-fluorophenyl.

In another aspect R^{a} is hydrogen.

In another aspect R^{b} is hydrogen or methyl.

In another aspect R^{c} is hydrogen.

In a further aspect R² is unsubstituted phenyl or naphthyl, mono-, di- or trisubstituted phenyl or naphthyl or mono-substituted heterocyclyl, the substituents being chosen from those described above.

Heterocyclyl is, for example, pyrimidinyl or pyridinyl. In a further aspect of the invention heterocyclyl is optionally substituted by C₁₋₄ alkyl or C₁₋₄ alkoxy.

In another aspect R² is hydrogen or phenyl optionally substituted by: halogen (for example fluoro), C₁₋₆ alkyl, C₁₋₆ alkoxy or (C₁₋₆ alkyl)C(O)NH.

In a further aspect the present invention provides a compound of formula (I) wherein X is O; R¹ is phenyl optionally substituted by halogen (for example chlorine) or C₁₋₄ alkyl (for example methyl); and R^{a}, R^{b}, R^{c} and R² is as defined above.

In a still further aspect the present invention provides a compound wherein Y is a bond or alkylene (optionally substituted by C₁₋₄ alkyl); R^{a} is hydrogen; and, R² is hydrogen, C₁₋₆ alkyl, phenyl (optionally substituted by halogen, C₁₋₄ alkyl, C₁₋₄ alkoxy or NHC(O)(C₁₋₄ alkyl)) or heterocyclyl (optionally substituted by halogen, C₁₋₄ alkyl or C₁₋₄ alkoxy).

In another aspect the present invention provides a compound wherein Y is phenylene (optionally substituted by halogen, C₁₋₄ alkyl or C₁₋₄ alkoxy) or heterocyclylene (optionally substituted by halogen, C₁₋₄ alkyl or C₁₋₄ alkoxy); R^{a} is hydrogen; and R² is hydrogen or C₁₋₄ alkyl.

When Z is tetrazolyl it is, for example, tetrazol-5-yl. In yet another aspect of the invention Z is CO₂R^{b} wherein R^{b} is hydrogen or C₁₋₄ alkyl (for example methyl).

The compounds of the invention can be prepared by adaptation of methods known in the art, by adaptation of the Examples given below or by using or adapting the methods in Scheme 1 {in which EDCI is ethyl dimethylaminopropyl carbodiimide; HOBT is 1-hydroxybenzotriazole hydrate; and DMAP is *N*,*N*-dimethylaminopyridine}.

A compound of formula (I), for example wherein R^{a} is hydrogen and Z is CO₂R^{b}, can be prepared by coupling a compound of formula (II): 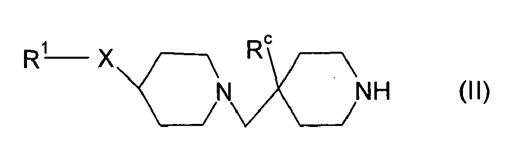 with a compound of formula (III): 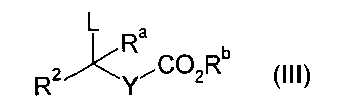 wherein L is a suitable leaving group (such as halogen (such as chloro or bromo), C₁₋₆ alkylsulfonyl (such as mesylate) or tosylate) and the coupling can be carried out in a suitable solvent (such as water or N,N-dimethylformamide) at ambient temperature.

Alternatively, a compound of formula (I), wherein R^{a} is hydrogen and Z is CO₂R^{b}, can be prepared by reductive amination of a compound (II) with a compound of formula (IV): 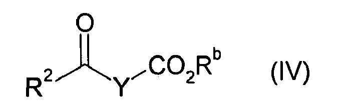 wherein R^{b} is C₁₋₄ alkyl, in the presence of NaBH(OAc)₃ and acetic acid, or NaBH₃CN in a suitable solvent (such as tetrahydrofuran), optionally followed by hydrolysis of the ester group.

Alternatively, a compound of formula (I), wherein Y is a bond, R^{a} and R^{b} are both hydrogen and Z is CO₂H, can be prepared by a three component coupling of a compound of formula (II) with compounds of formula (V) and (VI): 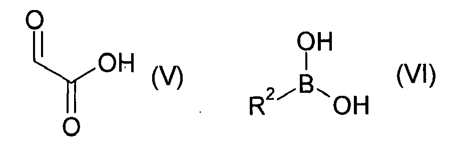 in a suitable solvent (such as a C₁₋₆ aliphatic alcohol (for example ethanol)) at a suitable elevated temperature (for example reflux; such as 60-100°C).

A compound of formula (II) can be prepared by deprotecting a compound of formula (VII): 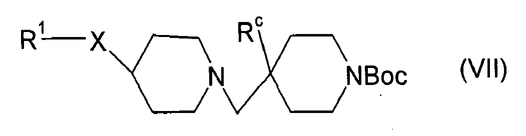 for example using trifluoroacetic acid in a suitable solvent (such as dichloromethane) or using a source of hydrogen chloride in a suitable solvent (such as dioxane).

A compound of formula (VII), wherein R^{c} is hydrogen, can be prepared by reacting a compound of formula (VIII): 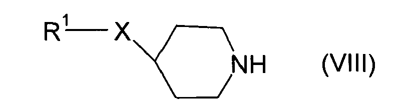 with a compound of formula (IX): 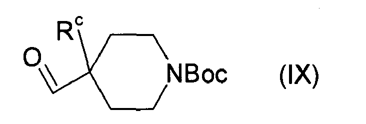 in the presence of NaBH(OAc)₃ and acetic acid, in a suitable solvent (such as tetrahydrofuran or dichloromethane).

A compound of formula (VII), wherein R^{c} is hydroxy, can be prepared by reacting a compound of formula (VIII) with a compound of formula (X): 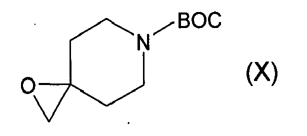 in a suitable solvent (such as a C₁₋₆ aliphatic alcohol, for example ethanol) at room temperature.

A compound of formula (I), wherein Y is a bond and Z is CO₂H, can be prepared by performing a nitrile hydrolysis on a compound of formula (XI): 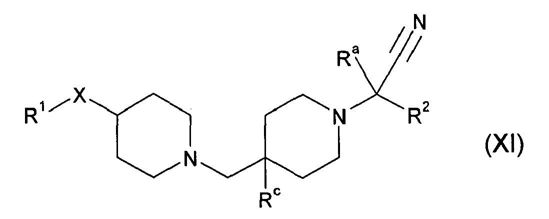 Such a hydrolysis can be carried out by refluxing a mixture of hydrochloric acid and ethanol; or by adding MeSO₃H, water and hydrochloric acid and then refluxing the mixture.

A compound of formula (XI) can be used to form a compound of formula (I) wherein Z is tetrazol-5-yl by reacting it with (CH₃)₃SiN₃ and (Bu₃Sn)₂O at an elevated temperature (for example in toluene at reflux).

A compound of formula (XI) can be reduced to form a compound of formula (XII): 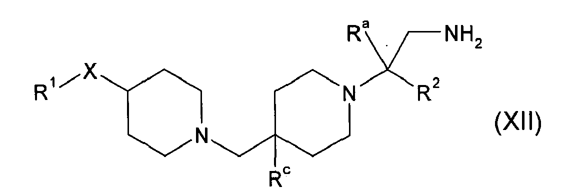 using sodium borohydride and cobalt (II) chloride in methanol. A compound of formula (XII) can then be reacted with triflic anhydride at a reduced temperature (for example -78°C in dichloromethane) to form the corresponding compound where Z is NHS(O)₂CF₃ .

A compound of formula (XI) can be prepared by reacting a compound of formula (II) with R^{a}R²C(O) and titanium isopropoxide (Ti(OiPr)₄), followed by Et₂AlCN. Longer chain variants of the compound of formula (XI) can be made by reacting a compound of formula (II) with: a compound Hal-(CH₂)ₙCN in the presence of a base (such as potassium carbonate) in acetone; or CH₂=CH-CN in the presence of a base (such as potassium carbonate) in acetone; wherein Hal is chlorine, bromine or iodine.

The preparation of various intermediates can be found in WO00/66559 and WO01/77101; alternatively they can be prepared by using or adapting literature methods.

Compounds of formula (III) to (IX) can be prepared by using or adapting methods described in the art. The preparation of various phenoxy piperidines is described in WO 01/77101.

A compound of formula (I), wherein Y is CHR^{d}; R^{d} is hydrogen, C₁₋₄ alkyl or phenyl; and Z is CO₂R^{b}, can be prepared by reacting a compound of formula (II) with an alkene of formula R²R^{a}C=CHR^{d}CO₂R^{b} in a suitable solvent, such as ethanol, at a suitable elevated temperature, such as 50-100°C.

A compound of formula (I), wherein R^{a} is hydrogen, Y is CH₂ and Z is CO₂R^{b}, can be prepared by reacting a compound of formula (II) with an alkyne of formula R²C≡CCO₂R^{b} in a suitable solvent, such as ethanol, at a suitable elevated temperature, such as 50-100°C; and then reducing the alkene product so formed (for example by catalytic hydrogenation).

A compound of formula (I), wherein R² and R^{a} are hydrogen, Y is phenylene (optionally substituted by halogen, hydroxy, C₁₋₄ alkyl or C₁₋₄ alkoxy) and Z is CO₂R^{b}, can be prepared by reacting a compound of formula (II) with a benzyl bromide of formula BrCH₂-Y-CO₂R^{b} in the presence of diisopropylethylamine (DIPEA), in a suitable solvent (such as acetonitrile) and at ambient temperature (such as in the range 10-30°C).

Alternatively, a compound of formula (I), wherein R² and R^{a} are hydrogen, Y is phenylene (optionally substituted by halogen, hydroxy, C₁₋₄ alkyl or C₁₋₄ alkoxy) and Z is CO₂R^{b}, can be prepared by reacting a compound of formula (II) with a benzaldehyde of formula (O)HC-Y-CO₂R^{b} wherein R^{b} is C₁₋₄ alkyl, in the presence of NaBH(OAc)₃ and acetic acid, in a suitable solvent (such as tetrahydrofuran), optionally followed by hydrolysis of the ester group.

Compounds of formula (I) wherein R², and R^{a} are both hydrogen; Y is CH₂; and Z is CO₂R^{b} can be prepared by a Michael addition of CH₂=CH-CO₂R^{b} on a compound of formula (II).

In another aspect the present invention provides processes for the preparation of compounds of formula (I) or (Ia).

The compounds of the invention have activity as pharmaceuticals, in particular as modulators of chemokine receptor (such as CCR3) activity, and may be used in the treatment of autoimmune, inflammatory, proliferative or hyperproliferative diseases, or immunologically-mediated diseases (including rejection of transplanted organs or tissues and Acquired Immunodeficiency Syndrome (AIDS)).

Examples of these conditions are:
(1) (the respiratory tract) obstructive diseases of airways including: chronic obstructive pulmonary disease (COPD) (such as irreversible COPD); asthma {such as bronchial, allergic, intrinsic, extrinsic or dust asthma, particularly chronic or inveterate asthma (for example late asthma or airways hyper-responsiveness)}; bronchitis {such as eosinophilic bronchitis}; acute, allergic, atrophic rhinitis or chronic rhinitis including rhinitis caseosa, hypertrophic rhinitis, rhinitis purulenta, rhinitis sicca or rhinitis medicamentosa; membranous rhinitis including croupous, fibrinous or pseudomembranous rhinitis or scrofulous rhinitis; seasonal rhinitis including rhinitis nervosa (hay fever) or vasomotor rhinitis; sarcoidosis; farmer's lung and related diseases; nasal polyposis; fibroid lung, idiopathic interstitial pneumonia, antitussive activity, treatment of chronic cough associated with inflammatory conditions of the airways or iatrogenic induced cough;
(2) (bone and joints) arthrides including rheumatic, infectious, autoimmune, seronegative spondyloarthropathies (such as ankylosing spondylitis, psoriatic arthritis or Reiter's disease), Behçet's disease, Sjogren's syndrome or systemic sclerosis;
(3) (skin and eyes) psoriasis, atopic dermatitis, contact dermatitis or other eczmatous dermitides, seborrhoetic dermatitis, lichen planus, phemphigus, bullous phemphigus, epidermolysis bullosa, urticaria, angiodermas, vasculitides erythemas, cutaneous eosinophilias, uveitis, alopecia areata, corneal ulcer or vernal conjunctivitis;
(4) (gastrointestinal tract) Coeliac disease, proctitis, eosinophilic gastro-enteritis, mastocytosis, Crohn's disease, ulcerative colitis, irritable bowel disease or food-related allergies which have effects remote from the gut (for example migraine, rhinitis or eczema);
(5) (Allograft rejection) acute and chronic following, for example, transplantation of kidney, heart, liver, lung, bone marrow, skin or cornea; or chronic graft versus host disease; and/or
(6) (other tissues or diseases) Alzheimer's disease, multiple sclerosis, atherosclerosis, Acquired Immunodeficiency Syndrome (AIDS), lupus disorders (such as lupus erythematosus or systemic lupus), erythematosus, Hashimoto's thyroiditis, myasthenia gravis, type I diabetes, nephrotic syndrome, eosinophilia fascitis, hyper IgE syndrome, leprosy (such as lepromatous leprosy), peridontal disease, Sezary syndrome, idiopathic thrombocytopenia pupura or disorders of the menstrual cycle.

The compounds of formula (I) or (Ia) or a pharmaceutically acceptable salt thereof or a solvate thereof, are also H1 antagonists (and can, therefore, be used in the treatment of allergic disorders); and may also be used to control a sign and/or symptom of what is commonly referred to as a cold (for example a sign and/or symptom of a common cold or influenza or other associated respiratory virus infection).

According to a further feature of the present invention there is provided a method for treating a chemokine mediated disease state (such as a CCR3 mediated disease state) in a mammal, such as man, suffering from, or at risk of, said disease state, which comprises administering to a mammal in need of such treatment a therapeutically effective amount of a compound of the formula (I) or (Ia) or a pharmaceutically acceptable salt thereof or a solvate thereof.

According to another feature of the present invention there is provided a method for antagonising H1 in a mammal, such as man, suffering from, or at risk of, an H1 mediated disease state, which comprises administering to a mammal in need of such treatment a therapeutically effective amount of a compound of the formula (I) or (Ia) or a pharmaceutically acceptable salt thereof or a solvate thereof.

According to yet another feature of the present invention there is provided a method for treating a sign and/or symptom of what is commonly referred to as a cold in a mammal, such as man, suffering from, or at risk of, said disease state, which comprises administering to a mammal in need of such treatment a therapeutically effective amount of a compound of the formula (I) or (Ia) or a pharmaceutically acceptable salt thereof or a solvate thereof.

The invention also provides a compound of the formula (I) or (Ia), or a pharmaceutically acceptable salt thereof or a solvate thereof, for use in therapy.

In another aspect the invention provides the use of a compound of formula (I) or (Ia), or a pharmaceutically acceptable salt thereof or a solvate thereof, in the manufacture of a medicament for use in therapy (for example modulating chemokine receptor activity (such as CCR3 receptor activity), antagonising H1 or treating a sign and/or symptom of what is commonly referred to as a cold).

The invention further provides the use of a compound of formula (I) or (Ia), or a pharmaceutically acceptable salt thereof, in the manufacture of a medicament for use in the treatment of:
(1) (the respiratory tract) obstructive diseases of airways including: chronic obstructive pulmonary disease (COPD) (such as irreversible COPD); asthma {such as bronchial, allergic, intrinsic, extrinsic or dust asthma, particularly chronic or inveterate asthma (for example late asthma or airways hyper-responsiveness)}; bronchitis {such as eosinophilic bronchitis}; acute, allergic, atrophic rhinitis or chronic rhinitis including rhinitis caseosa, hypertrophic rhinitis, rhinitis purulenta, rhinitis sicca or rhinitis medicamentosa; membranous rhinitis including croupous, fibrinous or pseudomembranous rhinitis or scrofulous rhinitis; seasonal rhinitis including rhinitis nervosa (hay fever) or vasomotor rhinitis; sarcoidosis; farmer's lung and related diseases; nasal polyposis; fibroid lung, idiopathic interstitial pneumonia, antitussive activity, treatment of chronic cough associated with inflammatory conditions of the airways or iatrogenic induced cough;
(2) (bone and joints) arthrides including rheumatic, infectious, autoimmune, seronegative spondyloarthropathies (such as ankylosing spondylitis, psoriatic arthritis or Reiter's disease), Behcet's disease, Sjogren's syndrome or systemic sclerosis;
(3) (skin and eyes) psoriasis, atopic dermatitis, contact dermatitis or other eczmatous dermitides, seborrhoetic dermatitis, lichen planus, phemphigus, bullous phemphigus, epidermolysis bullosa, urticaria, angiodermas, vasculitides erythemas, cutaneous eosinophilias, uveitis, alopecia areata, corneal ulcer or vernal conjunctivitis;
(4) (gastrointestinal tract) Coeliac disease, proctitis, eosinophilic gastro-enteritis, mastocytosis, Crohn's disease, ulcerative colitis, irritable bowel disease or food-related allergies which have effects remote from the gut (for example migraine, rhinitis or eczema);
(5) (Allograft rejection) acute and chronic following, for example, transplantation of kidney, heart, liver, lung, bone marrow, skin or cornea; or chronic graft versus host disease; and/or
(6) (other tissues or diseases) Alzheimer's disease, multiple sclerosis, atherosclerosis, Acquired Immunodeficiency Syndrome (AIDS), lupus disorders (such as lupus erythematosus or systemic lupus), erythematosus, Hashimoto's thyroiditis, myasthenia gravis, type I diabetes, nephrotic syndrome, eosinophilia fascitis, hyper IgE syndrome, leprosy (such as lepromatous leprosy), Peridontal disease, sezary syndrome, idiopathic thrombocytopenia pupura or disorders of the menstrual cycle;
in a mammal (for example man).

In a further aspect the invention provides a compound of formula (I) or (Ia), or a pharmaceutically acceptable salt thereof, for use in the treatment of asthma {such as bronchial, allergic, intrinsic, extrinsic or dust asthma, particularly chronic or inveterate asthma (for example late asthma or airways hyper-responsiveness)}; or rhinitis {including acute, allergic, atrophic or chronic rhinitis, such as rhinitis caseosa, hypertrophic rhinitis, rhinitis purulenta, rhinitis sicca or rhinitis medicamentosa; membranous rhinitis including croupous, fibrinous or pseudomembranous rhinitis or scrofulous rhinitis; seasonal rhinitis including rhinitis nervosa (hay fever) or vasomotor rhinitis}.

In a still further aspect a compound of formula (I) or (Ia), or a pharmaceutically acceptable salt thereof, is useful in the treatment of asthma.

The present invention also provides a the use of a compound of formula (I) or (Ia), or a pharmaceutically acceptable salt thereof, in the manufacture of a medicament for use in the treatment of asthma {such as bronchial, allergic, intrinsic, extrinsic or dust asthma, particularly chronic or inveterate asthma (for example late asthma or airways hyper-responsiveness)}; or rhinitis {including acute, allergic, atrophic or chronic rhinitis, such as rhinitis caseosa, hypertrophic rhinitis, rhinitis purulenta, rhinitis sicca or rhinitis medicamentosa; membranous rhinitis including croupous, fibrinous or pseudomembranous rhinitis or scrofulous rhinitis; seasonal rhinitis including rhinitis nervosa (hay fever) or vasomotor rhinitis}.

In order to use a compound of the invention, or a pharmaceutically acceptable salt thereof or solvate thereof, for the therapeutic treatment of a mammal, such as man, said ingredient is normally formulated in accordance with standard pharmaceutical practice as a pharmaceutical composition. Therefore in another aspect the present invention provides a pharmaceutical composition which comprises a compound of the formula (I) or (Ia), or a pharmaceutically acceptable salt thereof or a solvate thereof (active ingredient), and a pharmaceutically acceptable adjuvant, diluent or carrier.

In a further aspect the present invention provides a process for the preparation of said composition which comprises mixing active ingredient with a pharmaceutically acceptable adjuvant, diluent or carrier. Depending on the mode of administration, the pharmaceutical composition will, for example, comprise from 0.05 to 99 %w (per cent by weight), such as from 0.05 to 80 %w, for example from 0.10 to 70 %w, such as from 0.10 to 50 %w, of active ingredient, all percentages by weight being based on total composition.

The pharmaceutical compositions of this invention may be administered in standard manner for the disease condition that it is desired to treat, for example by topical (such as to the lung and/or airways or to the skin), oral, rectal or parenteral administration. For these purposes the compounds of this invention may be formulated by means known in the art. A suitable pharmaceutical composition of this invention is one suitable for oral administration in unit dosage form, for example a tablet or capsule which contains between 0.1 mg and 1 g of active ingredient.

Each patient may receive, for example, a dose of 0.01 mgkg⁻¹ to 100 mgkg⁻¹, such as in the range of 0.1 mgkg⁻¹ to 20 mgkg⁻¹, of the active ingredient administered, for example, 1 to 4 times per day.

The invention will now be illustrated by the following non-limiting examples in which, unless stated otherwise:
(i) when given, ¹H NMR data is quoted and is in the form of delta values for major diagnostic protons, given in parts per million (ppm) relative to tetramethylsilane (TMS) as an internal standard, determined at 300MHz or 400MHz using perdeuterio DMSO-D6 (CD₃SOCD₃) or CDCl₃ as the solvent unless otherwise stated;
(ii) mass spectra (MS) were run with an electron energy of 70 electron volts in the chemical ionisation (CI) mode using a direct exposure probe; where indicated ionisation was effected by electron impact (EI) or fast atom bombardment (FAB); where values for m/z are given, generally only ions which indicate the parent mass are reported, and unless otherwise stated the mass ion quoted is the positive mass ion - (M+H)⁺;
(iii) the title and sub-title compounds of the examples and methods were named using the index name program from Advanced Chemistry Development Inc;
(iv) unless stated otherwise, reverse phase HPLC was conducted using a Symmetry™, NovaPak™ or Xerra™ reverse phase silica column; and
(v) the following abbreviations are used:

| | | | | |
|---|---|---|---|---|
| Boc or BOC | tert-butoxycarbonyl | | DMSO | dimethylsulfoxide |
| HPLC | high pressure liquid chromatography | | aq | aqueous |
| DIPEA | Diisopropylethylamine | | THF | tetrahydrofuran |
| NMP | N-methylpyrrolidone | | MeCN | acetonitrile |

### INTERMEDIATE 1

### 4-(3,4-Dichlorophenoxy)-1-(4-piperidinylmethyl)-piperidine

### a) 1,1-Dimethylethyl 4-[[4-(3,4-dichlorophenoxy)-1-piperidinyl]methyl]-1-piperidinecarboxylate

4-(3,4-Dichlorophenoxy)piperidine (1.27 g) was dissolved in THF (20 mL); acetic acid (0.5 mL) and 1,1-dimethylethyl 4-formyl-1-piperidinecarboxylate (1.43 g) were added to the solution. The reaction mixture was stirred at room temperature for 30 min then sodium triacetoxyborohydride (1.53 g) was added and the mixture was stirred at room temperature overnight. The reaction mixture was poured into 2M sodium hydroxide solution (50 mL) and product was extracted with ether. The ether was washed with brine, dried, filtered and evaporated. Crude material was purified by flash chromatography (eluting with 979 : 20 : 1 dichloromethane : methanol : aqueous ammonia) to give the subtitle compound (2.15 g).
MS 443/445 [M+H]⁺ (ES+)
¹H NMR δ_{(CDCl3)} 1.06 (2H, ddd), 1.45 (9H, s), 1.61 - 1.82 (5H, m), 1.92 - 1.98 (2H, m), 2.16 - 2.27 (4H, m), 2.65 - 2.73 (4H, m), 4.08 (2H, d), 4.25 (1H, dq), 6.75 (1H, dd), 6.99 (1H, d), 7.30 (1H, d)

### b) 4-(3,4-dichlorophenoxy)-1-(4-piperidinylmethyl)-piperidine

1,1-Dimethylethyl 4-[[4-(3,4-dichlorophenoxy)-1-piperidinyl]methyl]-1-piperidinecarboxylate (1.0 g) was added to a mixture of 20% TFA in dichloromethane (20 mL) and the mixture was stirred at room temperature for 1h. Solvent was removed by evaporation and 2M sodium hydroxide solution (25 mL) was added to the residue. Product was extracted with ethyl acetate. The organic phase was washed with brine, dried, filtered and evaporated to give the title compound (0.5 g).
MS 343/345 [M+H]⁺ (ES+)
¹H NMR δ_{(CDCl3)} 1.10 (2H, qd), 1.60 (1H, qquintet), 1.73 - 1.83 (4H, m), 1.90 - 2.01 (2H, m), 2.16 - 2.26 (4H, m), 2.55 - 2.70 (4H, m), 3.09 (2H, d), 4.24 (1H, dquintet), 6.75 (1H, dd), 6.99 (1H, d), 7.27 (1H, d)

The following intermediates were prepared analogously from the appropriate aryloxy piperidine:

| Intermediate | Name | M+H | ¹H NMR |
|---|---|---|---|
| 2 | 4-(2,4-Dichloro-3-methylphenoxy)-1-(4-piperidinylmethyl)-piperidine | 357/359 | δ_{(CDCl3)} 1.13 - 1.27 (2H, m), 1.57 - 1.70 (1H, m), 1.76 - 2.00 (2H, m), 2.16 - 2.32 (4H, m), 2.46 (3H, s), 2.60 - 2.99 (8H, m), 3.16 (2H, d), 4.31 (1H, quintet), 6.75 (1H, d), 7.18 (1H, d) |
| 3 | 4-(4-Chloro-2-methylphenoxy)-1-(4-piperidinylmethyl)-piperidine | 323/325 | δ_{(CDCl3)} 1.08 - 1.21 (2H, m), 1.56 - 1.68 (1H, m), 1.73 - 1.86 (4H, m), 1.90 - 1.99 (2H, m), 2.16 - 2.31 (7H, m), 2.57 - 2.69 (4H, m), 3.12 (2H, d), 4.23 - 4.31 (1H, m), 6.74 (1H, d), 7.06 (1H, dd), 7.11 (1H, d) |
| 4 | 4-(2,4-Dichlorophenoxy)-1-(4-piperidinylmethyl)-piperidine | 343/345 | |
| 5 | 4-(3,4-Dichloro-2-methylphenoxy)-1-(4-piperidinylmethyl)-piperidine | 357/359 | δ_{(CD3OD)} 1.10 - 1.22 (2H, m), 1.66 - 1.85 (5H, m), 1.94 - 2.04 (2H, m), 2.22 (2H, d), 2.31 (3H, s), 2.32 - 2.41 (2H, m), 2.59 - 2.72 (4H, m), 3.08 (2H, d), 4.38 - 4.46 (1H, m), 6.91 (1H, d), 7.27 (1H, d) |

### INTERMEDIATE 6

### 4-(3-Chloro-4-fluoro-phenoxy)-piperidine

DEAD (0.43 mL) was added to a solution of triphenylphosphine (0.72 g), 3-chloro-4-fluorophenol (0.403 g) and 4-hydroxy-piperidine-1-carboxylic acid tert-butyl ester (0.5 g) in THF at RT. The resulting mixture was stirred overnight, HCl in dioxan (2 mL of 4M) was added and the mixture stirred at RT overnight. The mixture was then evaporated to dryness and triethylamine (5 mL) was added. The mixture was evaporated and the residue was dissolved in methanol (10 mL), placed onto a SCX cartridge (Varian, 10 g, SCX cartridge available from International Sorbent Technology Isolute® Flash SCX-2) and eluted: first with methanol then with 10%NH₃ in methanol. The basic fractions were combined and evaporated to give the product as an oil (0.6 g).
¹H NMR δ_{(DMSO-D6)} 1.34 - 1.46 (2H, m), 1.83 - 1.91 (2H, m), 2.53 - 2.59 (2H, m), 2.87 - 2.96 (2H, m), 3.22 - 3.39 (1H, m), 4.39 (1H, septet), 6.92 - 6.98 (1H, m), 7.17 - 7.20 (1H, m), 7.30 (1H, t).

The following intermediate was prepared in similar manner to intermediate 6

| Intermediate | name | M+H |
|---|---|---|
| 7 | 4-(3,4-Dichloro-2-methylphenoxy)-piperidine | 260/262 |

### INTERMEDIATE 8

### 4-[[4-(3,4-Dichlorophenoxy)-1-piperidinyl]methyl]- 4-piperidinol

### a) 1,1-Dimethylethyl 4-[[4-(3,4-dichlorophenoxy)-1-piperidinyl]methyl]-4-hydroxy-1-piperidinecarboxylate

A solution of 4-(3,4-dichlorophenoxy)-piperidine (5.2 g) and 1,1-dimethylethyl 1-oxa-6-azaspiro[2.5]octane-6-carboxylate (4.1 g) in ethanol (50 mL) was stirred at room temperature for 18 hours and then at 60°C for 18 hours. The solvent was evaporated to leave 9.5 g of a pale yellow oil. Flash chromatography (dichloromethane then dichloromethane : 7M ammonia in methanol 95:5) gave the subtitle compound (8.48 g).
MS [M+H]⁺ (ES+) 459/461
¹H NMR δ_{(CDCl3)} 1.35 - 1.63 (4H, m), 1.46 (9H, s), 1.73 - 1.86 (2H, m), 1.89 - 2.01 (2H, m), 2.34 (2H, s), 2.49 - 2.59 (2H, m), 2.79 - 2.89 (2H, m), 3.07 - 3.24 (2H, m), 3.79 - 3.93 (2H, m), 4.22 - 4.32 (1H, m), 6.75 (1H, dd), 6.99 (1H, d), 7.30 (1H, d)

### b) 4-[[4-(3,4-Dichlorophenoxy)-1-piperidinyl]methyl]-4-piperidinol

To a solution of 1,1-dimethylethyl 4-[[4-(3,4-dichlorophenoxy)-1-piperidinyl]methyl]-4-hydroxy-1-piperidinecarboxylate (5 g) in dichloromethane (50 mL) was added trifluoroacetic acid (5 mL) and the solution was stirred for 12 hours. Sodium hydroxide solution (1M) was added to give an alkaline solution, this was then extracted thrice with dichloromethane. The pooled organic phase was subsequently washed with water, dried, filtered and evaporated to give the title compound (3.5 g).
MS [M+H]⁺ (ES+) 359/361
¹H NMR δ_{(CDCl3)} 1.57 - 1.66 (4H, m), 1.69 - 1.84 (2H, m), 1.93 - 2.04 (2H, m), 2.36 (2H, s), 2.47 - 2.58 (2H, m), 2.82 - 2.92 (4H, m), 2.96 - 3.07 (2H, m), 4.32 - 4.41 (1H, m), 6.89 (1H, dd), 7.09 (1H, d), 7.38 (1H, d)

### INTERMEDIATE 9

### 4-[[4-(3,4-Dichlorophenoxy)-1-piperidinyl] methyl]-1,2-cyclopentanediol

### a) 4-(3,4-Dichlorophenoxy)-1-[1-oxo-2-(2-propenyl)-4-pentenyl]-piperidine

A solution of 4-(3,4-dichlorophenoxy)-piperidine (5.25 g) in dichloromethane (80 mL) was added to a solution of EDCI (2.45 g), HOBT (1.77 g) and DMAP (0.44 g) in dichloromethane (100 mL). A solution of 2-(2-propenyl)-4-pentenoic acid (1.81 g) in dichloromethane (5 mL) was added and the solution was stirred for 60 h. The reaction mixture was poured onto water. The mixture was separated and the aqueous phase was extracted twice with dichloromethane. The organic phases were washed with brine, dried, filtered and evaporated to give an oil. Chromatography of the oil (eluting dichloromethane, then 49:1 dichloromethane : methanol) gave the subtitle compound (3.40 g).
MS [M+H]⁺ (ES+) 368/342
¹H NMR δ_{(CDCl3)} 5.69 - 5.83 (2H, m), 5.00 - 5.11 (4H, m), 4.46 - 4.52 (1H, m), 3.62 - 3.85 (3H, m), 3.43 - 3.53 (1H, m), 2.76 - 2.87 (1H, m), 2.37 - 2.47 (2H, m), 2.17 - 2.27 (2H, m), 1.70 - 1.99 (4H, m), 6.77 (1H, dd), 7.01 (1H, d), 7.33 (1H, d)

### b) 1-(3-Cyclopenten-1-ylcarbonyl)-4-(3,4-dichlorophenoxy)-piperidine

Nitrogen was bubbled through solution of 4-(3,4-dichlorophenoxy)-1-[1-oxo-2-(2-propenyl)-4-pentenyl]-piperidine (1.45 g) in dichloromethane (20 mL) for 10 min. with sonication (cleaning bath). Grubbs' catalyst (89 mg) was added and the solution was stirred for 16 h. Water was added and the phases were separated. The aqueous phase was extracted twice with dichloromethane, the organics were dried, filtered and concentrated to give the subtitle compound as a green oil (1.60 g)
MS [M+H]⁺ (ES+) 340/342
¹H NMR δ_{(CDCL3)} 4.47 - 4.53 (1H, m), 5.67 (2H, s), 7.33 (1H, d), 6.78 (1H, dd), 7.02 (1H, d), 3.62 - 3.84 (3H, m), 3.44 - 3.52 (1H, m), 3.33 (1H, d), 2.68 - 2.77 (2H, m), 2.54 - 2.64 (2H, m), 1.88 - 1.99 (2H, m), 1.73 - 1.86 (2H, m)

### c) cis and trans 4-(3,4-Dichlorophenoxy)-1-[(3,4-dihydroxycyclopentyl)carbonyl]-piperidine

1-(3-Cyclopenten-1-ylcarbonyl)-4-(3,4-dichlorophenoxy)-piperidine (1.45 g) was dissolved in acetone (30 mL) and water (20 mL). Osmium tetroxide (1 mL of 2.5% solution in t-butanol) was added and the solution was stirred for 5 days. The reaction mixture was poured onto a solution of sodium metabisulfite. The mixture was extracted thrice with dichloromethane, the organic extracts were washed with brine, dried, filtered and evaporated to give an oil. Chromatography (eluting dichloromethane : methanol 24:1 to 37 : 3) gave the title compound as two compounds (0.31 g and 0.71 g).
MS [M+H]⁺ (ES+) 374/376
Minor isomer ¹H NMR δ_{(CDCl3)} 1.79 - 1.98 (6H, m), 2.12 - 2.22 (2H, m), 3.23 (1H, tt), 3.49 - 3.56 (1H, m), 3.65 - 3.79 (3H, m), 3.93 (1H, d), 3.99 - 4.08 (3H, m), 4.53 (1H, tt), 6.77 (1H, dd), 7.02 (1H, d), 7.34 (1H, d)
Major isomer ¹H NMR δ_{(CDCl3)} 1.73 - 1.86 (2H, m), 1.86 - 2.00 (4H, m), 2.07 - 2.16 (2H, m), 2.50 - 2.60 (2H, m), 3.39 (1H, tt), 3.42 - 3.48 (1H, m), 3.61 - 3.78 (3H, m), 4.22 - 4.27 (2H, m), 4.47 - 4.53 (1H, m), 6.77 (1H, dd), 7.01 (1H, d), 7.33 (1H, d)

### d) 4-[[4-(3,4-Dichlorophenoxy)-1-piperidinyl]methyl]-1,2-cyclopentanediol

Borane solution (16 mL of 1M in THF) was added to 4-(3,4-dichlorophenoxy)-1-[(3,4-dihydroxycyclopentyl)carbonyl]-piperidine (major isomer, 0.71 g) and the resulting solution was heated to reflux for 90 min. Methanol (10 mL) was added and the mixture was heated under reflux for 1 h. The solvent was removed and the residue was loaded onto an SCX2 cartridge with methanol. Washing with methanol followed by elution with 0.7M ammonia in methanol gave the title compound as a viscous oil containing solvent.
MS [M+H]⁺ (ES+) 360/362

The following intermediates were prepared analogously from the appropriate aryloxy piperidine as a mixture of isomers:

| Intermediate | Name | M+H | ¹H NMR |
|---|---|---|---|
| 10 | 4-[[4-(2,4-Dichloro-3-methylphenoxy)-1-piperidinyl]methyl]-1,2-cyclopentanediol | 374/376 | δ_{(CD3OD)} 1.28 - 1.37 (0.66H, m), 1.37 - 1.48 (1.34H, m), 1.69 - 1.81 (4H, m), 1.84 - 2.09 (3H, m), 2.27 (2H, d), 2.35 (3H, s), 2.36 - 2.53 (2H, m), 2.61 - 2.76 (2H, m), 3.78 - 3.85 (0.66H, m), 3.90 - 3.96 (1.34H, m), 4.32 - 4.43 (1H, m), 6.85 (1H, d), 7.16 (1H, d) |
| 11 | 4-[[4-(3,4-Dichloro-2-methylphenoxy)-1-piperidinyl]methyl]-1,2-cyclopentanediol | 374/376 | δ_{(CD3OD)} 1.28 - 1.36 (0.66H, m), 1.39 - 1.48 (1.34H, m), 1.68 - 1.80 (4H, m), 1.86 - 1.98 (3H, m), 2.22 (3H, s), 2.25 (2H, d), 2.29 - 2.50 (2H, m), 2.60 - 2.70 (2H, m), 3.78 - 3.84 (0.66H, m), 3.89 - 3.95 (1.34H, m), 4.29 - 4.38 (1H, m), 6.82 (1H, d), 7.18 (1H, d) |

### INTERMEDIATE 12

### 4-(3,4-Dichlorophenoxy)-1-(4-piperidinylmethyl)-piperidineacetonitrile

4-(3,4-Dichlorophenoxy)-1-(4-piperidinylmethyl)-piperidine (0.5 g), bromoacetonitrile (0.21 g), diisopropylethylamine (0.36 mL) and dimethylformamide (3 mL) were stirred together at room temperature, under nitro gen, for 4 hours. The mixture was poured into water (50 mL), extracted into ethyl acetate (3 x 50 mL), washed with brine (50 mL), dried, filtered and evaporated. Flash chromatography (29:1 dichloromethane: methanol) gave the title compound as a solid (363 mg).
MS [M+H]⁺ (APCI+) 382/384
¹H NMR δ_{(CDCL3)} 1.24 (2H, qd), 1.45 - 1.55 (1H, m), 1.73 - 1.85 (4H, m), 1.92 - 2.00 (2H, m), 2.19 (2H, d), 2.20 - 2.27 (2H, m), 2.34 (2H, td), 2.63 - 2.71 (2H, m), 2.80 (2H, d), 3.53 (2H, s), 4.21 - 4.28 (1H, m), 6.75 (1H, dd), 6.99 (1H, d), 7.30 (1H, d)

The following intermediates were prepared analogously from the appropriate aryloxy piperidine:

| Intermediate | Name | M+H | ¹H NMR |
|---|---|---|---|
| 13 | 4-[[4-(3,4-Dichloro-2-methylphenoxy)-1-piperidinyl]methyl]-1-piperidineacetonitrile | 396/398 | |

### INTERMEDIATE 14

### 4-[[4-(3,4-Dichlorophenoxy)-1-piperidinyl]methyl]-1-piperidinepropanenitrile

4-(3,4-Dichlorophenoxy)-1-(4-piperidinylmethyl)-piperidine (0.85 g), acrylonitrile (0.24 mL), diisopropylethylamine (0.72 mL) and dimethylformamide (6 mL) were stirred together at room temperature, under nitrogen, for 24 hours. The mixture was poured into water (50 mL), extracted into ethyl acetate (3 x 50 mL), washed with brine (50 mL), dried, filtered and evaporated. Flash chromatography (19:1 dichloromethane: methanol) gave the title compound as a solid (116 mg).
MS [M+H]⁺ (APCI+) 396/398
¹H NMR δ_{(CD3OD)} 1.06 - 1.23 (2H, m), 1.40 - 1.53 (1H, m), 1.60 - 1.75 (4H, m), 1.84 - 1.93 (2H, m), 1.95 - 2.06 (2H, m), 2.11 - 2.17 (2H, m), 2.17 - 2.30 (2H, m), 2.43 - 2.70 (6H, m), 2.76 - 2.95 (2H, m), 4.20 - 4.40 (1H, m), 6.78 (1H, dd), 6.99 (1H, d), 7.28 (1H, d)

### INTERMEDIATE 15

### 4-[[4-(3,4-Dichlorophenoxy)-1-piperidinyl]methyl]-1-piperidineethanamine

4-[[4-(3,4-Dichlorophenoxy)-1-piperidinyl]methyl]-1-piperidineacetonitrile (0.43 g) and cobalt (II) chloride (0.3 g) in methanol (20 mL) were cooled to 5 °C, under nitrogen, and sodium borohydride (0.43 g) was added portionwise. The mixture was stirred at 5 °C for 40 minutes then poured into 2N aqueous sodium hydroxide solution (50 mL), extracted into ethyl acetate (3 x 50 mL), dried, filtered and evaporated to give the title compound (0.43 g).
¹H NMR δ_{(CDCl3)} 1.08 - 1.28 (3H, m), 1.50 - 1.80 (6H, m), 1.88 - 2.02 (3H, m), 2.04 - 2.22 (4H, m), 2.45 (1H, s), 2.56 - 2.73 (3H, m), 2.89 (2H, m), 3.07 - 3.10 (1H, d), 4.23 (1H, m), 6.74 - 6.76 (1H, d), 6.99 (1H, s), 7.26 -7.31 (1H, t)

The following intermediates were prepared analogously from the appropriate nitrile:

| Intermediate | Name | M+H | ¹H NMR |
|---|---|---|---|
| 16 | 4-[[4-(3,4-Dichloro-2-methylphenoxy)-1-piperidinyl]methyl]-1-piperidineethanamine | | δ(_{CDCl3}) 1.09 - 1.26 (3H, m), 1.62 - 1.85 (6H, m), 1.88 - 2.01 (3H, m), 2.16 - 2.18 (2H, d), 2.21 - 2.30 (2H, m), 2.32 (3H, s), 2.39 - 2.44 (1H, m), 2.58 -2.71 (3H, m), 2.75 -2.98 (2H, t), 3.03 - 3.16 (1H, d), 4.62 (1H, m), 6.70 - 6.73 (1H, d), 7.19 -7.22 (1H, d) |
| 17 | 4-[[4-(3,4-Dichlorophenoxy)-1-piperidinyl]methyl]-1-piperidinepropanamine | | δ_{(CDCl3)} 1.02 - 1.23 (2H, m), 1.40 - 1.83 (11H, m), 1.85 - 1.94 (3H, m), 2.08 - 2.20 (4H, m), 2.65 (3H, m), 2.93 -3.20 (2H, m), 4.23 (1H, m), 6.73 - 6.76 (1H, d), 6.99 (1H, s), 7.26 -7.31 (1H, t) |

### INTERMEDIATE 18

### 1-Methylethyl 3-formyl-2-pyridinecarboxylate

1-Methylethyl 3-(hydroxymethyl)-2-pyridinecarboxylate (1.2 g) was dissolved in dichloromethane (20 mL) and to the solution was added Dess-Martin periodinane (3.0 g). The reaction mixture was stirred, under nitrogen, at room temperature, for 1 h. Sodium thiosulphate (10 g) was added to a saturated aqueous solution of sodium bicarbonate (25 mL) and this mixture was added to the reaction mixture. Ether (25 mL) was then added and the mixture was stirred rapidly for 5min. The mixture was separated, the aqueous phase was extracted with ether (2x20 mL). 2M HCl (10 mL) was added to the combined ether extracts. The aqueous phase was removed, basified by careful addition of solid sodium bicarbonate and extracted with ether. This ether was dried (MgSO₄), filtered and concentrated *in vacuo* to give the title compound as a colourless oil (0.87 g).
¹H NMR δ_{(DMSO)} 1.35 (6H, d), 5.24 (1H, quintet), 7.80 (1H, dd), 8.31 (1H, dd), 8.86 (1H, dd), 10.29 (1H, s)

### INTERMEDIATE 19

### Methyl 4-(bromomethyl)-3-fluoro- benzoate

Methyl 3-fluoro-4-methyl benzoate (0.97 g), N-bromosuccinimide (1.13 g) and azobisisobutyronitrile (0.02 g) were added to carbon tetrachloride (2 mL) and the mixture was heated under reflux, whilst being irradiated with a 100W lamp, for 6h. The reaction mixture was concentrated *in vacuo* and the residue was partitioned between ethyl acetate and 1 M hydrochloric acid. The organic phase was washed with brine, dried (MgSO₄) and filtered to give a crude yellow oil which was purified by flash chromatography, eluting with 5% ethyl acetate in isohexane to give the title compound as a colourless oil (0.63 g). ¹H NMR δ_{(CDCl3)} 3.93 (3H, s), 4.52 (2H, d), 7.47 (1H, t), 7.73 (1H, dd), 7.81 (1H, dd)

### INTERMEDIATE 20

### Methyl 2-(bromomethyl)-5-fluoro benzoate

Prepared following the method for Intermediate 15.
¹H NMR δ_{(CDCl3)} 3.95 (3H, s), 4.93 (2H, s), 7.20 (1H, ddd), 7.46 (1H, dd), 7.67 (1H, dd)

### INTERMEDIATE 21

### Ethyl 4-[(4-hydroxy-1-piperidinyl)methyl]-α-phenyl-1-piperidineacetate

### a) Ethyl 4-(hydroxymethyl)-α-phenyl-1-piperidineacetate

Ethyl α-bromobenzeneacetate (2.43 g) was dissolved in acetone (20 mL). A suspension of 4-hydroxymethylpiperidine (1.15 g) in acetone (5 mL) was added followed by potassium carbonate (2.60 g). The mixture was stirred for 16 h, filtered and concentrated to an oil. Chromatography (isohexane : ethyl acetate 1:1, then 3:7) gave the subtitle compound as an oil (2.23 g).
MS [M+H]⁺ (ES+) 278
¹H NMR δ_{(CDCl3)} 1.21 (3H, t), 1.32 (1H, td), 1.41 (1H, td), 1.46 - 1.57 (1H, m), 1.63 - 1.69 (1H, m), 1.70 - 1.77 (1H, m), 1.89 (1H, td), 2.16 (1H, td), 2.76 - 2.81 (1H, m), 2.98 - 3.04 (1H, m), 3.50 (2H, d), 3.99 (1H, s), 4.09 - 4.24 (2H, m), 7.30 - 7.37 (3H, m), 7.42 - 7.46 (2H, m)

### b) Ethyl 4-formyl-α-phenyl-1-piperidineacetate

DMSO (1.1 mL) was dissolved in dichloromethane (15 mL) and cooled below - 60 °C. Oxalyl chloride (0.9 mL) in dichloromethane (5 mL) was added dropwise maintining the temperature below -57°C. The solution was stirred for 15 min. then ethyl 4-(hydroxymethyl)-α-phenyl-1-piperidineacetate (2.23 g) dissolved in dichloromethane (6 mL) was added dropwise and the solution was stirred for 30 min. Triethylamine (4 mL) was added and the reaction mixture was allowed to warm to ambient temperature. Water was added, the phases were separated, the aqueous was extracted twice with dichloromethane and the organic phases were washed with brine, dried, filtered and concentrated to give the subtitle compound.
MS [M+H]⁺ (ES+) 276
¹H NMR δ_{(CDCl3)} 1.21 (3H, t), 1.64 - 1.81 (2H, m), 1.82 - 1.95 (1H, m), 2.11 (1H, td), 2.19 - 2.34 (2H, m), 2.70 - 2.80 (2H, m), 2.81 - 2.90 (1H, m), 4.04 (1H, s), 4.07 - 4.25 (2H, m), 7.30 - 7.38 (3H, m), 7.39 - 7.44 (2H, m), 9.63 (1H, d)

### c) Ethyl 4-[(4-hydroxy-1-piperidinyl)methyl]-α-phenyl-1-piperidineacetate

4-Hydroxypiperidine (0.81 g) and ethyl 4-formyl-α-phenyl-1-piperidineacetate (2.14 g) were suspended in THF (10 mL). Acetic acid (0.5 mL) was added followed by sodium triacetoxyborohydride (1.68 g) and then THF (6 mL). The suspension was stirred overnight, then sodium bicarbonate solution was added and the mixture was stirred for 5 min. The suspension was extracted thrice with ethyl acetate, the organic phases were washed with brine, dried, filtered and evaporated. Chromatography of the residue (dichloromethane : methanol : triethylamine 90:9: 1) gave the subtitle compound as an oil (2.14 g).
MS [M+H]⁺ (ES+) 361
¹H NMR δ_{(CDCl3)} 1.20 (3H, td), 1.33 (2H, qd), 1.42 - 1.49 (1H, m), 1.49 - 1.57 (2H, m), 1.69 - 1.76 (2H, m), 1.81 - 1.89 (3H, m), 2.00 - 2.12 (3H, m), 2.14 (2H, d), 2.58 - 2.78 (4H, m), 2.93 - 2.98 (1H, m), 3.61 - 3.70 (1H, m), 3.97 (1H, s), 4.07 - 4.23 (2H, m), 7.29 - 7.36 (3H, m), 7.41 - 7.45 (2H, m).

### EXAMPLE 1

This Example illustrates the preparation of 4-[[4-(3,4-dichlorophenoxy)-1-piperidinyl]methyl]-α-phenyl-1-piperidineacetic acid.

4-{[4-(3,4-Dichlorophenoxy)piperidin-1-yl]methyl}piperidine (0.5 g), and benzene boronic acid (0.2 g) were dissolved in ethanol (3 mL); glyoxylic acid (0.2 mL of a 50% solution in water) was added to the solution and the reaction mixture was heated in a microwave oven at 100°C for 5min. The resultant solution was purified by HPLC (gradient 95% - 5% aqueous ammonium acetate, 5% - 95% acetonitrile) to give the title compound (0.1 g).
MS [M+H]⁺ (ES+) 477/479
¹H NMR δ (CDCl₃) 1.53 - 1.77 (4H, m), 1.79 - 1.94 (4H, m), 2.14 - 2.25 (4H, m), 2.41 (1H, t), 2.54 - 2.64 (2H, m), 2.75 (1H, t), 3.38 (1H, d), 3.58 - 3.70 (2H, m), 4.15 - 4.23 (1H, m), 4.47 (1H, s), 6.71 (1H, dd), 6.96 (1H, d), 7.25 (1H, d), 7.32 - 7.38 (3H, m), 7.49 - 7.58 (2H, m).

Examples 2-19 (see Table I below) were made using the method of Example 1.

### EXAMPLE 20

This Example illustrates the preparation of 4-[[4-(2,5-dichlorophenoxy)-1-piperidinyl]methyl]-α-phenyl-1-piperidineacetic acid

Ethyl 4-[(4-hydroxy-1-piperidinyl)methyl]-α-phenyl-1-piperidineacetate (0.135 g) was dissolved in NMP (3 mL). 1,4-Dichloro-2-fluorobenzene (0.2 mL) and potassium t-butoxide (56 mg) were added and the solution was heated to 50 °C for 40 h. The solution was cooled to ambient temperature and few drops of aqueous sodium hydroxide solution were added. The mixture was stirred for 60 h, then acetic acid (few drops) was added and the solvent was distilled. The residue was purified by HPLC (0.2% aqueous ammonia : acetonitrile; gradient 95:5 to 50:50) to give the title compound (21 mg).
MS [M+H]⁺ (ES+) 477/479
¹H NMR δ_{(CD3OD)} 1.45 (1H, q), 1.68 - 1.96 (9H, m), 2.16 - 2.21 (2H, m), 2.25 - 2.34 (2H, m), 2.57 - 2.65 (3H, m), 2.80 - 2.93 (2H, m), 4.29 - 4.36 (1H, m), 4.38 - 4.44 (1H, m), 6.83 (1H, dd), 7.02 (1H, d), 7.23 (1H, d), 7.32 - 7.36 (3H, m), 7.44 - 7.49 (2H, m)

Example 21 (see Table I below) was made using the method of Example 20

### EXAMPLE 22

This Example illustrates the preparation of methyl 4-[[4-(3,4-dichlorophenoxy)-1-piperidinyl]methyl]-α-phenyl-piperidineacetate.

4-(3,4-Dichlorophenoxy)-1-(4-piperidinylmethyl)-piperidine (0.30 g) and methyl-α-bromobenzeneacetate (0.22 g) were dissolved in acetone (20 mL) and potassium carbonate (0.13 g) was added. The reaction mixture was stirred at room temperature for 16 h. The suspension was filtered and the filtrate was evaporated. The residue was chromatographed eluting with ethyl acetate : methanol : triethylamine (20 : 1 : 0.001) to give the title compound (0.24 g).
MS [M+H]⁺ (ES+) 491/493
¹H NMR δ_{(CD3OD)} 1.22 (1H, qd), 1.34 (2H, qd), 1.50 - 1.59 (1H, m), 1.66 (1H, d), 1.70 - 1.80 (3H, m), 1.88 (1H, td), 1.93 - 2.02 (2H, m), 2.14 (1H, td), 2.22 (2H, d), 2.25 - 2.33 (1H, m), 2.65 - 2.73 (3H, m), 2.95 - 3.01 (1H, m), 3.68 (3H, s), 3.98 (1H, s), 4.37 (1H, septet), 6.87 (1H, dd), 7.08 (1H, d), 7.31 - 7.38 (4H, m), 7.42 (2H, dd)

### EXAMPLES 23 & 24

This Example illustrates the preparation of (*R*)-methyl 4-[[4-(3,4-dichlorophenoxy)-1-piperidinyl]methyl]-α-phenyl-piperidineacetate and (*S*)-methyl 4-[[4-(3,4-dichlorophenoxy)-1-piperidinyl]methyl]-α-phenyl-piperidineacetate.

Racemic methyl 4-[[4-(3,4-dichlorophenoxy)-1-piperidinyl]methyl]-α-phenyl-piperidineacetate (360 mg) was dissolved in isohexane : isopropanol (9:1) and was chromatographed on a Chiralpak AD column eluting isohexane : isopropanol (9: 1) to give the 2 isomers.

First eluting isomer (50 mg); MS [M+H]⁺ (ES+) 491/493. Retention time (chiralpak AD column (4.6 x 250 mm), maintained at 10 °C, flow rate 1 mL/min 95:5 isohexane : isopropanol containing 0.1 % diethylamine) 7.2 minutes.

Second eluting isomer (30 mg); MS [M+H]⁺ (ES+) 491/493. Retention time (chiralpak AD column (4.6 x 250 mm), maintained at 10 °C, flow rate 1 mL/min 95:5 isohexane : isopropanol containing 0.1 % diethylamine) 8.9 minutes

### EXAMPLE 25

This Example illustrates the preparation of (*R*)-4-[[4-(3,4-dichlorophenoxy)-1-piperidinyl]niethyl]-a-phenyl-piperidineacetic acid.

Methyl (*R*)-4-[[4-(3,4-dichlorophenoxy)-1-piperidinyl]methyl]-α-phenyl-1-piperidineacetate (45 mg) was dissolved in aq. HCl (6M, 10 mL) and heated at 80° C for 22hrs. It was dried on a rotary evaporator, redissolved in MeOH and purified by HPLC (gradient 95% - 50% aqueous ammonium acetate, 5% - 50% acetonitrile) to give the title compound (14.1 mg).
MS [M+H]⁺ (ES+) 477/479
¹H NMR δ_{(CD3OD + NaOD)} 1.27 - 1.37 (2H, m), 1.45 - 1.62 (2H, m), 1.72 - 2.06 (8H, m), 2.31 - 2.36 (2H, m), 2.36 - 2.45 (2H, m), 2.72 - 2.80 (2H, m), 2.97 (1H, t), 4.37 - 4.46 (2H, m), 6.88 (1H, dd), 7.09 (1H, d), 7.37 (1H, d), 7.42 - 7.46 (3H, m), 7.54 - 7.58 (2H, m)

Example 26 (see Table I below) was made using the method of Example 25

### EXAMPLE 27

This Example illustrates the preparation of (R)-methyl 4-[[4-(3,4-dichlorophenoxy)-1-piperidinyl]methyl]-α-phenyl-piperidineacetate

4-[[4-(3,4-Dichlorophenoxy)-1-piperidinyl]methyl]-1,2-cyclopentanediol (Intermediate 9, major isomer, 230 mg) was dissolved in dichloromethane (5 mL). Sodium carbonate (225 mg) was added and the resulting suspension was cooled in ice-water. Lead tetraacetate (310 mg) was added in small portions over 15 min. A suspension of (*R*)-phenylglycine methyl ester hydrochloride (129 mg) and sodium triacetoxyborohydride (300 mg) in THF (10 mL) was prepared in a separate flask. To this suspension was added acetic acid (50 µL) and triethylamine (100 µL) then the suspension was sonicated (cleaning bath) for 5 min. 40 min after the completion of the addition of lead tetraacetate to the diol the resulting suspension was filtered through a plug of cotton wool into the aminoester suspension, followed by a rinse of THF (3 mL). Additional acetic acid (50 µL) and triethylamine (100 µL) were added to the reaction mixture which was then stirred overnight.

Aqueous sodium bicarbonate was added to the reaction mixture which was then extracted thrice with ethyl acetate. The extracts were combined, washed with brine, dried, filtered and evaporated. The residue was purified by chromatography (39:1 ethyl acetate : methanol) to give the title compound (157 mg).
MS [M+H]⁺ (ES+) 491/493
¹H NMR δ(_{CDCl3}) 1.24 (1H, qd), 1.33 (1H, qd), 1.41 - 1.52 (1H, m), 1.70 - 1.80 (3H, m), 1.85 (1H, td), 1.90 - 1.98 (2H, m), 2.12 (1H, td), 2.16 - 2.25 (5H, m), 2.62 - 2.69 (2H, m), 2.75 (1H, d), 2.94 (1H, d), 3.69 (3H, s), 4.01 (1H, s), 4.19 - 4.26 (1H, m), 6.74 (1H, dd), 6.98 (1H, d), 7.29 (1H, d), 7.31 - 7.36 (3H, m), 7.40 - 7.44 (2H, m)

### Examples 28 - 33 (see Table I below) were made using the method of Example 27

### EXAMPLE 34

This Example illustrates the preparation of (*R*)-4-[[4-(3,4-dichlorophenoxy)-1-piperidinyl]methyl]-α-phenyl-piperidineacetic acid dihydrochloride

(R)-Methyl 4-[[4-(3,4-dichlorophenoxy)-1-piperidinyl]methyl]-α-phenyl-piperidineacetate (150 mg) was suspended in 6M hydrochloric acid (20 mL) and heated to 80 °C for 22 h. The crystalline solid formed was collected and dried *in vacuo* to give the title compound (100 mg).
m. pt. 294-297 C
MS [M+H]⁺ (ES+) 477/479 ppp
¹H NMR δ_{(CD3OD)} 1.43 - 1.59 (1H, m), 1.66 (1H, q), 1.86 - 2.02 (2H, m), 2.05 - 2.29 (5H, m), 2.78 - 2.93 (1H, m), 2.98 - 3.18 (12H, m), 3.37 - 3.45 (2H, m), 3.61 (1H, d), 3.74 - 3.88 (1H, m), 4.47 - 4.57 (0H, m), 4.67 - 4.72 (1H, m), 5.00 - 5.12 (1H, m), 6.83 - 6.91 (1H, m), 7.09 - 7.16 (1H, m), 7.31 - 7.36 (1H, m)

Examples 35 - 40 (see Table I below) were made using the method of Example 25.

### EXAMPLE 41

This Example illustrates the preparation of 1-[4-[[4-(3,4-dichlorophenoxy)-1-piperidinyl]methyl]-1-piperidinyl]-2,3-dihydro-1*H*-indene-1-carboxylic acid

4-[[4-(3,4-Dichlorophenoxy)-1-piperidinyl]methyl]-1,2-cyclopentanediol (0.20 g) was dissolved in dichloromethane (10 mL) and sodium carbonate (0.206 g) was added. The suspension was cooled to 0°C. Lead tetraacetate (0.248 g) was added over 20 minutes. The mixture was stirred for 40 min at 0 C.

The suspension was filtered through a plug of cotton wool into a solution of 1-amino-2,3-dihydro-1*H*-indene-1-carboxylic acid (0.098 g), hydrochloric acid (0.1 mL), triethylamine (0.1 mL) and methanol (10 mL). Sodium cyanoborohydride (0.052 g) was added and the reaction mixture was stirred for 16 h at room temperature. The solvents were evaporated and the residue was redissolved in acetonitrile/water and AcOH was added. This was purified by HPLC (5% MeCN/95%NH₄OAc aq (0.1%) gradient to 50% MeCN/50%NH₄OAc) to give title compound (93 mg).
MS [M+H]⁺ (ES+) 503/505.
¹H NMR δ_{(CD3OD + NaOD)} 1.17 - 1.27 (1H, m), 1.29 - 1.41 (2H, m), 1.46 - 1.54 (1H, m), 1.54 - 1.70 (3H, m), 1.83 - 1.93 (3H, m), 1.97 - 2.24 (6H, m), 2.42 - 2.52 (2H, m), 2.55 - 2.65 (2H, m), 2.71 - 2.80 (1H, m), 2.87 - 3.05 (2H, m), 4.22 - 4.31 (1H, m), 6.74 - 6.80 (1H, m), 6.97 - 7.03 (4H, m), 7.27 (1H, d), 7.44 (1H, d)

### EXAMPLE 42

This Example illustrates the preparation of methyl 2-[(4-{[4-(3,4-dichlorophenoxy)piperidin-1-yl]methyl}piperidin-1-yl)methyl]benzoate

4-(3,4-Dichlorophenoxy)-1-(piperidin-4-ylmethyl)piperidine (0.5 g) was dissolved in acetonitrile (2 mL) and to the solution was added methyl 2-(bromomethyl)benzoate (0.56 g) and DIPEA (0.25 mL). The reaction mixture was stirred at room temperature overnight then concentrated by evaporation under reduced pressure. The residue was partitioned between ethyl acetate and water, the organic phase was washed with brine, dried (MgSO₄), filtered and concentrated to give an oil. This was purified by chromatography eluting with 5% methanol in dichloromethane then by HPLC (25% MeCN/75%NH₄OAc aq (0.1%) gradient to 95% MeCN/5%NH₄OAc) to give the title compound as an oil 0.4 g.
MS [M+H]⁺ (ES+) 491/493.
¹H NMR δ_{(CDCl3)} 1.10 - 1.24 (2H, m), 1.46 (1H, qd), 1.63 - 2.05 (8H, m), 2.15 - 2.28 (4H, m), 2.62 - 2.71 (2H, m), 2.76 - 2.82 (2H, m), 3.74 (2H, s), 3.87 (3H, s), 4.23 (1H, quintet), 6.74 (1H, dd), 6.99 (1H, d), 7.25 - 7.32 (2H, m), 7.37 - 7.46 (2H, m), 7.68 (1H, d).

Example 43 (see Table I below) were made using the method of Example 42.

### EXAMPLE 44

This Example illustrates the preparation of methyl 2-[[4-[[4-(2,4-dichlorophenoxy)-1-piperidinyl]methyl]-1-piperidinyl]methyl]-5-fluoro-benzoate

4-(2,4-Dichlorophenoxy)-1-(4-piperidinylmethyl)-piperidine dihydrochloride (0.26 g) was added to acetonitrile (3 mL) and treated with triethylamine (0.26 mL). After stirring for 5min, methyl 2-(bromomethyl)-5-fluoro benzoate (0.15 g) was added and the reaction mixture was stirred at room temperature overnight. The reaction mixture was concentrated *in vacuo* and crude product was purified by flash chromatography, eluting with 2% methanol and 0.1 % triethylamine in dichloromethane, giving the title compound contaminated with triethylamine hydrochloride.
MS [M+H]⁺ (ES+) 509/511

Examples 45, 48-50 were prepared following the method of example 44.

### EXAMPLE 46

This Example illustrates the preparation of 1-methylethyl- 3-[[4-[[4-(3,4-dichlorophenoxy)-1-piperidinyl]methyl]-1-piperidinyl]methyl]-2-pyridinecarboxylate

4-(3,4-Dichlorophenoxy)-1-(4-piperidinylmethyl)-piperidine (0.3 g) and 1-methylethyl-3-formyl-2-pyridinecarboxylate (0.17 g) were added to a mixture of THF (3 mL) and acetic acid (0.5 mL). The mixture was stirred at room temperature for 5min then sodium triacetoxyborohydride (0.28 g) was added. The mixture was stirred overnight then poured into a saturated solution of sodium bicarbonate. The product was extracted with ethyl acetate, the organic phase was washed with brine, dried (MgSO₄), filtered and concentrated *in vacuo.* The residue was purified by flash chromatography, eluting with 3% methanol and 0.1% triethylamine in dichloromethane, giving the title compound as a clear oil (0.24 g).
¹H NMR δ_{(CD3OD)} 1.13 - 1.28 (2H, m), 1.43 (6H, d), 1.50 - 1.65 (1H, m), 1.69 - 1.83 (4H, m), 1.96 - 2.11 (4H, m), 2.23 (2H, d), 2.27 - 2.37 (2H, m), 2.67 - 2.84 (4H, m), 3.72 (2H, s), 4.35 - 4.45 (1H, m), 5.26 (1H, t), 6.90 (1H, dd), 7.11 (1H, d), 7.39 (1H, d), 7.52 (1H, dd), 7.93 (1H, dd), 8.49 (1H, dd)

Examples 47, 60 - 66 (Table I below) were prepared following the method of Example 46.

### EXAMPLES 51-59

Examples 51-59 (Table I below) were made from Examples 42-50 by the methods of Example 77 (LiOH, Examples 51, 53, 54, 57, 58, 59), Example 25 (HCl, Examples 55, 56) or Example 90 (KOTMS, Example 52).

### EXAMPLE 67

This Example illustrates the preparation of methyl 4-[[4-(3,4-dichlorophenoxy)-1-piperidinyl]methyl]-1-piperidineacetate

To a stirred solution of 4-(3,4-dichlorophenoxy)-1-(4-piperidinylmethyl)-piperidine (0.23 g) and DIPEA (0.164 mL) in DMF at RT was added methyl bromoacetate (0.076 mL). The reaction was heated at 60°C for 16 h. Saturated sodium bicarbonate solution (30 mL) was then added to the cooled solution and the product was extracted into ethyl acetate (3 x 20 mL). The combined organics were washed with brine (10 mL) and then dried, filtered and evaporated to leave a colourless oil (0.135 g).
MS [M+H]⁺ (ES+) 415/417

Examples 68-72 (see Table I) were prepared analogously to Example 67 from the appropriate amine.

### EXAMPLE 73

This Example illustrates the preparation of methyl (2R)-2-(4-{[4-(3,4-dichlorophenoxy)piperidin-1-yl]methyl}piperidin-1-yl)propanoate

Diethyl ether (10 mL) and dimethylformamide (2 mL) were added to 4-(3,4-dichlorophenoxy)-1-(piperidin-4-ylmethyl)piperidine (0.32 g) and the mixture was sonicated (cleaning bath) until it became clear. Methyl (2*S*)-2-bromopropanoate (0.16 g) and triethylamine (0.6 mL) were added and the mixture was stirred at room temperature overnight. The reaction mixture was poured into water and was extracted with diethyl ether. The diethyl ether was washed with brine, dried (MgSO₄), filtered and concentrated under reduced pressure to give an oil. Crude product was purified by chromatography, eluting with 95 : 5 : 0.1 dichloromethane : methanol : aqueous ammonia to give the title compound as an oil (0.25 g).
MS [M+H]⁺ (ES+) 429/431

Examples 74-76 (see Table I) were prepared analogously to Example 73.

### EXAMPLE 77

This Example illustrates the preparation of 4-[[4-(3,4-dichlorophenoxy)-1-piperidinyl]methyl]-1-piperidineacetic acid.

Methyl 4-[[4-(3,4-dichlorophenoxy)-1-piperidinyl]methyl]-1-piperidineacetate (0.135 g) and lithium hydroxide (0.136 g) in 3 :1 methanol/water (2 mL) was stirred at RT for 16 h. The reaction mixture was acidified to pH 4 with acetic acid and purified by HPLC (10% MeCN/90%NH₄OAc aq (0.1%) gradient to 70% MeCN/30%NH₄OAc) to provide the title compound as a white solid (0.030 g).
MS [M+H]⁺ (ES+) 401/403.
¹H NMR δ_{(CD3OD)} 1.52 (2H, qd), 1.72 - 1.92 (3H, m), 1.98 - 2.09 (4H, m), 2.34 (2H, d), 2.38 - 2.45 (2H, m), 2.72 - 2.83 (2H, m), 3.01 (2H, td), 3.56 - 3.67 (4H, m), 4.35 - 4.49 (1H, m), 6.90 (1H, dd), 7.11 (1H, d), 7.39 (1H, d).

Examples 78-86 (see Table I) were prepared analogously to Example 77 from the appropriate ester.

### EXAMPLE 87

This Example illustrates the preparation of methyl 4-[[4-(3,4-dichlorophenoxy)-1-piperidinyl]methyl]-α,α-dimethyl-1-piperidine propanoate

To a stirred solution of 4-{[4-(3,4-dichlorophenoxy)piperidin-1-yl]methyl}piperidine (0.175 g) and 2,2-dimethyl-3-oxopropanoic acid methyl ester (80 mg) in THF (0.5 mL) was added sodium triacetoxyborohydride (162 mg) and acetic acid (0.041 mL). The reaction mixture was stirred at room temperature overnight. Saturated sodium bicarbonate solution (30 mL) was added and the product was extracted into ethyl acetate (3 x 20 mL). The combined organics were washed with brine (10 mL) and dried (MgSO₄), filtered and evaporated to leave an oil (0.17 g). A portion (0.080 g) was purified by HPLC (5% MeCN/95%NH₄OAc aq (0.1%) gradient to 5% MeCN/95%NH₄OAc) to give the title compound as an oil (0.012 g).
MS [M+H]⁺ (ES+) 457/459.
¹H NMR δ_{(CDCl3)} 1.15 (6H, s), 1.16 (1H, qd), 1.34 - 1.45 (1H, m), 1.58 - 1.62 (2H, m), 1.62 - 1.66 (2H, m), 1.71 - 1.82 (2H, m), 1.90 - 2.00 (2H, m), 2.07 - 2.16 (3H, m), 2.16 - 2.26 (2H, m), 2.45 (2H, s), 2.60 - 2.70 (2H, m), 2.70 - 2.77 (2H, m), 3.65 (3H, s), 4.18 - 4.27 (1H, m), 6.74 (1H, dd), 6.99 (1H, d), 7.30 (1H, d).

Examples 88 & 89 (see Table I) were prepared analogously to Example 87 from the appropriate amines.

### EXAMPLE 90

This Example illustrates the preparation of 4-[[4-(3,4-dichlorophenoxy)-1-piperidinyl]methyl]-α,α-dimethyl-1-piperidine propanoic acid.

To a stirred solution of methyl 4-[[4-(3,4-dichlorophenoxy)-1-piperidinyl]methyl]-α,α-dimethyl-1-piperidine propanoate (0.080 g) in THF (1 mL) at RT was added potassium trimethylsilanolate (27 mg). After 16 h the reaction mixture was incomplete and further potassium trimethylsilanolate (27 mg) was added. After a further 1 h the reaction solvent was evaporated and the residue was redissolved in acetonitrile and purified by HPLC (5% MeCN/95%NH₄OAc aq (0.1 %) gradient to 60% MeCN/40%NH₄OAc) to give the title compound (0.036 g).
MS [M+H]⁺ (ES+) 443/445.
¹H NMR δ_{(CD3OD)} 1.22 (6H, s), 1.47 (2H, q), 1.68 - 1.81 (2H, m), 1.79 - 1.88 (1H, m), 1.93 - 2.05 (4H, m), 2.27 (2H, d), 2.33 (2H, t), 2.67 - 2.76 (2H, m), 2.95 - 3.02 (2H, m), 3.04 (2H, s), 3.45 - 3.52 (2H, m), 4.33 - 4.42 (1H, m), 6.87 (1H, dd), 7.08 (1H, d), 7.36 (1H, d).

Example 91 & 92 (Table I) were prepared analogously to Example 90 from the appropriate esters

### EXAMPLE 93

This Example illustrates the preparation of 4-[[4-(3,4-Dichlorophenoxy)-1-piperidinyl]methyl]-1-piperidine propanoic acid dihydrochloride

To a stirred solution of 4-{[4-(3,4-dichlorophenoxy)piperidin-1-yl]methyl}piperidine (0.175 g) in isopropanol (0.4 mL) at RT was added acrylic acid (0.038 mL). After 16 h the reaction mixture was purified by HPLC (5% MeCN/95%NH₄OAc aq (0.1%) gradient to 50% MeCN/50%NH₄OAc). Treatment of the product with 2 M HCl at 40 °C for 15 min followed by evaporation left a yellow solid. This was triturated with diethyl ether (3 mL) and the residual solid was partially dissolved in 4 : 1 dichloromethane/methanol. The supernatant was evaporated to provide the title compound as a solid (0.014 g).
MS [M+H]⁺ (ES+) 415/417.
¹H NMR δ_{(D2O)} 1.63 (2H, qd), 1.91 - 2.05 (1H, m), 2.09 - 2.21 (2H, m), 2.26 (2H, d), 2.29 - 2.36 (1H, m), 2.40 (1H, d), 2.87 (2H, t), 3.08 (2H, t), 3.14 - 3.22 (2H, m), 3.29 - 3.40 (2H, m), 3.44 (2H, t), 3.52 (1H, d), 3.64 - 3.79 (3H, m), 4.61 - 4.70 (1H, m), 6.96 - 7.03 (1H, m), 7.24 - 7.29 (1H, m), 7.50 (1H, d).

### EXAMPLE 94

This Example illustrates the preparation of 4-[[4-(3,4-dichlorophenoxy)-1-piperidinyl]methyl]-1-piperidinebutanoic acid.

4-(3,4-Dichlorophenoxy)-1-(4-piperidinylmethyl)-piperidine (0.20 g) and methyl 4-bromo-butanoate (0.10 g) were dissolved in acetone (20 mL) and potassium carbonate (0.08 g) was added. The reaction mixture was stirred for 16 h at room temperature. The reaction mixture was filtered and the solvents were evaporated to give the title compound (18 mg).
MS [M+H]⁺ (ES+) 443/445

Example 95 & 96 (Table I) wre prepared analogously to Example 94 from the appropriate halo esters.

Examples 97-99 (Table I) were prepared from the appropriate esters by the method of Example 25.

### EXAMPLE 100

This Example illustrates the preparation of 4-(3,4-dichlorophenoxy)-1-[[1-(2*H-*tetrazol-5-ylmethyl)-4-piperidinyl]methyl]-piperidine

4-[[4-(3,4-Dichlorophenoxy)-1-piperidinyl]methyl]-1-piperidineacetonitrile (0.26 g), azidotrimethylsilane (0.5 mL), dibutyltin oxide (0.17 g) and toluene (10 mL) were heated together at 110 °C, in a sealed tube, for 20 hours, cooled and evaporated. The residue was dissolved in methanol, and filtered through reverse-phase silica to remove the tin by-products. The product was further purified by reverse-phase HPLC (25% MeCN/75%NH₄OAc aq (0.1%) gradient to 95% MeCN/5%NH₄OAc). This gave the title compound as a solid (0.24 g).
MS [M+H]⁺ (APCI+) 425/427.
¹H NMR δ_{(CD3OD)} 1.16 - 1.38 (2H, m), 1.71 - 1.84 (5H, m), 1.91 - 2.05 (2H, m), 2.37 - 2.49 (2H, m), 2.50 - 2.69 (4H, m), 2.79 - 2.98 (2H, m), 3.20 - 3.25 (2H, m), 4.12 (2H, s), 4.33 - 4.46 (1H, m), 6.81 (1H, dd), 7.04 (1H, d), 7.29 (1H, d)

Example 101 (Table I) was prepared analogously to Example 100 from the appropriate nitrile.

### EXAMPLE 102

This Example illustrates the preparation of *N*-[2-[4-[[4-(3,4-dichlorophenoxy)-1-piperidinyl]methyl]-1-piperidinyl]ethyl]-1,1,1-trifluoro-methanesulfonamide

4-[[4-(3,4-Dichlorophenoxy)-1-piperidinyl]methyl]-1-piperidineethanamine (0.28 g) in dichloromethane (25 mL) was cooled to - 78°C under nitrogen, and triflic anhydride 0.35 mL) was added dropwise. After 5 minutes the reaction was quenched with excess aqueous ammonia solution, warmed to room temperature, and evaporated. The product was purified by reverse-phase HPLC (25% MeCN/75%NH₄OAc aq (0.1%) gradient to 95% MeCN/5%NH₄OAc). This gave the title compound as a solid (0.08 g).
MS [M+H]⁺ (APCI+) 518/520.
¹H NMR δ_{(CD3OD)} 1.25 (2H, dd), 1.54 - 1.72 (3H, m), 1.79 (2H, d), 1.85 - 1.95 (2H, m), 2.18 (2H, d), 2.25 (2H, t), 2.35 (2H, td), 2.57 - 2.74 (4H, m), 3.11 (2H, d), 3.25 (2H, t), 4.19 - 4.43 (1H, m), 6.79 (1H, dd), 7.00 (1H, d), 7.28 (1H, d)

Examples 103 and 104 (Table I) were prepared analogously to Example 102 from the appropriate amines.

**TABLE I**

| Example | Name | M+H | ¹H NMR |
|---|---|---|---|
| 2 | 4-[[4-(2,4-Dichloro-3-methylphenoxy)-1-piperidinyl]methyl]-α-phenyl-1-piperidineacetic acid | 491/493 | δ_{(CDCl3)} 1.56 - 1.72 (3H, m), 1.83 - 1.97 (6H, m), 2.23 (2H, d), 2.30 - 2.39 (2H, m), 2.45 (3H, s), 2.50 - 2.52 (1H, m), 2.62 - 2.68 (2H, m), 2.76 - 2.84 (1H, m), 3.39 (1H, d), 3.71 (2H, d), 4.32 (1H, s), 4.57 (1H, s), 6.71 (1H, d), 7.17 (1H, d), 7.36 - 7.38 (3H, m), 7.53 - 7.56 (2H, m) |
| 3 | 4-[[4-(3,4-Dichlorophenoxy)-1-piperidinyl]methyl]-α-(4-fluorophenyl)-1-piperidineacetic acid | 495/497 | δ_{(CDCl3)} 1.56 - 1.78 (5H, m), 1.84 - 1.98 (4H, m), 2.18 - 2.32 (4H, m), 2.37 - 2.53 (1H, m), 2.57 - 2.67 (2H, m), 2.72 - 2.84 (1H, m), 3.36 - 3.43 (1H, m), 3 .64 - 3.72 (1H, m), 4.19 - 4.26 (1H, m), 4.54 (1H, s), 6.72 (1H, dd), 6.96 (1H, d), 7.07 (2H, t), 7.24 - 7.32 (1H, m), 7.55 (2H, dd) |
| 4 | 4-[[4-(3;4-Dichlorophenoxy)-1-piperidinyl]methyl]-α-(2-methoxyphenyl)-1-piperidineacetic acid | 507/509 | δ_{(CDCl3)} 1.55 - 1.79 (5H, m), 1.86 - 2.00 (4H, m), 2.16 - 2.27 (4H, m), 2.39 - 2.74 (4H, m), 2.87 (1H, t), 3.37 (1H, d), 3.69 - 3.78 (1H, m), 3.87 (3H, s), 4.18 - 4.26 (1H, m), 5.03 (1H, s), 6.72 (1H, dd), 6.91 - 7.03 (3H, m), 7.25 - 7.31 (1H, m), 7.37 (1H, t), 7.51 (1H, d) |
| 5 | 4-[[4-(3,4-Dichlorophenoxy)-1-piperidinyl] methyl]-α-(2-methylphenyl)-1-piperidineacetic acid | 491/493 | δ_{(CDCl3)} 1.47 - 1.75 (4H, m), 1.80 - 1.95 (5H, m), 2.12 - 2.23 (4H, m), 2.43 - 2.66 (6H, m), 2.76 - 2.90 (1H, m), 3.39 (1H, d), 3.49 (1H, s), 3.84 - 3.96 (1H, m), 4.14 - 4.25 (1H, m), 4.76 (1H, s), 6.72 (1H, dd), 6.96 (1H, d), 7.16 - 7.32 (4H, m), 7.78 (1H, d) |
| 6 | 4-[[4-(3,4-Dichlorophenoxy)-1-piperidinyl]methyl]-α-(4-methylphenyl)-1-piperidineacetic acid | 491/493 | δ_{(CDCl3)} 1.55 - 1.79 (5H, m), 1.81 - 1.96 (4H, m), 2.14 - 2.25 (4H, m), 2.35 (3H, s), 2.43 - 2.73 (4H, m), 2.76 - 2.87 (1H, m), 3.47 (1H, d), 3.68 - 3.77 (1H, m), 4.15 - 4.25 (1H, m), 4.54 (1H, s), 6.72 (1H, dd), 6.96 (1H, d), 7.18 (2H, d), 7.26 - 7.31 (1H, m), 7.42 (2H, d) |
| 7 | 4-[[4-(2,4-Dichloro-3-methylphenoxy)-1-piperidinyl]methyl]-α-(2-methoxyphenyl)-1-piperidineacetic acid | 521/523 | δ_{(CDCl3}) 1.55 - 1.68 (2H, m), 1.74 - 2.00 (5H, m), 2.16 - 2.28 (4H, m), 2.45 (3H, s), 2.57 - 2.90 (6H, m), 3.41 (1H, d), 3.66 - 3.77 (1H, m), 3.87 (3H, s), 4.24 - 4.35 (1H, m), 5.09 (1H, s), 6.72 (1H, d), 6.92 - 7.02 (2H, m), 7.17 (1H, d), 7.36 (1H, dd), 7.53 (1H, d) |
| 8 | 4-[[4-(2,4-Dichloro-3-methylphenoxy)-1-piperidinyl]methyl]-α-(2-methylphenyl)-1-piperidineacetic acid | 505/507 | δ_{(CDCl3)} 1.73 - 1.96 (8H, m), 2.14 - 2.28 (5H, m), 2.45 (3H, s), 2.53 (3H, s), 2.57 - 2.66 (4H, m), 2.75 - 2.86 (1H, m), 3.36 (1H, d), 3.80 - 3.91 (1H, m), 4.24 - 4.32 (1H, m), 4.73 (1H, s), 6.71 (1H, d), 7.14 - 7.24 (4H, m), 7.77 (1H, d) |
| 9 | 4-[[4-(2,4-Dichloro-3-methylphenoxy)-1-piperidinyl]methyl]-α-(4-methylphenyl)-1-piperidineacetic acid | 505/507 | δ_{(CDCl3)} 1.55 - 1.94 (9H, m), 2.14 - 2.27 (4H, m), 2.35 (3H, s), 2.45 (3H, s), 2.52 - 2.82 (5H, m), 3.46 (1H, d), 3.64 - 3.73 (1H, m), 4.24 - 4.32 (1H, m), 4.47 (1H, s), 6.71 (1H, d), 7.17 (3H, d), 7.43 (2H, d) |
| 10 | 4-[[4-(4-Chloro-2-methylphenoxy)-1-piperidinyl]methyl]-α-(2-methoxyphenyl)-1-piperidineacetic acid | 487/489 | δ_{(CDCl3)} 1.58 - 1.65 (2H, m), 1.70 - 1.80 (4H, m), 1.85 - 1.95 (4H, m), 2.15 - 2.26 (7H, m), 2.46 - 2.74 (3H, m), 2.80 - 2.91 (1H, m), 3.42 (1H, d), 3.68 - 3.77 (1H, m), 3.87 (3H, s), 4.19 - 4.28 (1H, m), 5.09 (1H, s), 6.70 (1H, d), 6.91 - 7.11 (4H, m), 7.36 (1H, dd), 7.5 (1H, d) |
| 11 | 4-[[4-(4-Chloro-2-methylphenoxy)-1-piperidinyl]methyl]-α-(2-methylphenyl)-1-piperidineacetic acid | 471/473 | δ_{(CDCl3)} 1.48 - 1.95 (11H, m), 2.13 (3H, s), 2.25 (2H, t), 2.46 - 2.90 (8H, m), 3.36 (1H, d), 3.83 - 3.93 (1H, m), 4.19 - 4.28 (1H, m), 4.78 (1H, s), 6.70 (1H, d), 7.02 - 7.11 (2H, m), 7.15 - 7.25 (3H, m), 7.74 (1H, d) |
| 12 | α-[4-[[4-(3,4-Dichlorophenoxy)-1-piperidinyl]methyl]-1-piperidinyl]-2,4-dimethoxy-5-pyrimidineacetic acid | 539/541 | δ_{(CDCl3)} 1.48 - 2.80 (19H, m), 3.27 - 3.35 (1H, m), 3.51 (3H, s), 3.99 (3H, s), 4.29 - 4.37 (1H, m), 4.55 (1H, s), 6.73 (1H, dd), 6.98 (1H, d), 7.31 (1H, d), 8.06 (1H, s) |
| 13 | 4-[[4-(4-Chloro-2-methylphenoxy)-1-piperidinyl]methyl]-α-(4-methylphenyl)-1-piperidineacetic acid | 471/473 | δ_{(CDCl3)} 1.56 - 1.79 (5H, m), 1.83 - 1.94 (4H, m), 2.13 - 2.27 (7H, m), 2.35 (3H, s), 2.44 - 2.95 (5H, m), 3.48 (1H, d), 3.71 (1H, d), 4.19 - 4.28 (1H, m), 4.50 (1H, s), 6.70 (1H, d), 7.03 - 7.11 (2H, m), 7.18 (2H, d), 7.43 (2H, d) |
| 14 | α-[3-(Acetylamino)phenyl]-[4-(3,4-dichlorophenoxy)-1-piperidiny]methyl]-1-piperidineacetic acid | 534/536 | δ_{(CDCl3)} 1.46 - 1.64 (2H, m), 1.68 - 1.83 (3H, m), 1.87 - 1.97 (2H, m), 2.05 - 2.25 (7H, m), 2.36 - 2.77 (8H, m), 3.33 - 3.68 (2H, m), 4.22 (1H, s), 4.41 (1H, s), 6.73 (1H, dd), 6.96 - 7.02 (2H, m), 7.22 - 7.33 (2H, m), 7.49 (1H, s), 8.09 (1H, d) |
| 15 | 4-[[4-(2,4-Dichloro-3-methylphenoxy)-1-piperidinyl]methyl]-α-(4-fluorophenyl)-1-piperidineacetic acid | 509/511 | δ_{(CDCl3)} 1.54 - 2.47 (18H, m), 2.61 - 2.72 (2H, m), 3.28 - 3.35 (1H, m), 3.54 - 3.61 (1H, m), 4.29 - 4.42 (2H, m), 6.71 (1H, d), 7.05 (2H, t), 7.17 (1H, d), 7.54 (2H, dd) |
| 16 | 4-[[4-(2,4-Dichlorophenoxy)-1-piperidinyl]methyl]-α-phenyl-1-piperidineacetic acid | 477/479 | δ_{(CD3OD)} 1.48 - 1.61 (2H, m), 1.75 - 1.89 (4H, m), 1.90 - 2.04 (4H, m), 2.28 (2H, d), 2.33 - 2.41 (2H, m), 2.67 - 2.77 (3H, m), 2.96 (2H, t), 4.37 - 4.50 (2H, m), 7.07 (1H, d), 7.22 (1H, dd), 7.39 (1H, d), 7.42 - 7.46 (3H, m), 7.54 - 7.58 (2H, m) |
| 17 | 4-[[4-(3,4-Dichlorophenoxy)-1-piperidinyl]methyl]-4-hydroxy-α-phenyl-1-piperidineacetic acid | 493/495 | δ_{(CD3OD)} 1.64 - 1.82 (4H, m), 1.85 - 2.03 (3H, m), 2.40 (2H, s), 2.49 - 2.57 (2H, m), 2.81 - 2.90 (3H, m), 2.98 - 3.16 (2H, m), 3.51 - 3.72 (2H, m), 4.32 - 4.39 (1H, m), 4.52 (1H, s), 6.87 (1H, dd), 7.08 (1H, d), 7.37 (1H, d), 7.42 - 7.47 (3H, m), 7.55 - 7.60 (2H, m) |
| 18 | 4-[[4-(3,4-Dichlorophenoxy)-1-piperidinyl]methyl]-4-hydroxy-α-(4-methylphenyl)-1-piperidineacetic acid | 507/509 | δ_{(CD3OD)} 1.64 - 1.81 (4H, m), 1.82 - 2.02 (4H, m), 2.36 (3H, s), 2.40 (2H, s), 2.49 - 2.57 (2H, m), 2.81 - 2.89 (2H, m), 2.92 - 3.06 (2H, m), 3.62 3.75 (2H, m), 4.32 - 4.39 (1H, m), 4.47 (1H, s), 6.87 (1H, dd), 7.08 (1H, d), 7.26 (2H, d), 7.36 (1H, d), 7.44 (2H, d) |
| 19 | 4-[[4-(3,4-Dichlorophenoxy)-1-piperidinyl]methyl]-4-hydroxy-α-(2-methoxyphenyl)-1-piperidineacetic acid | 523/525 | δ_{(CD3OD)} 1.65 - 1.80 (4H, m), 1.93 - 2.04 (4H, m), 2.40 (2H, s), 2.48 - 2.57 (2H, m), 2.81 - 2.89 (2H, m), 2.94 - 3.06 (2H, m), 3.67 - 3.77 (2H, m), 3.91 (3H, s), 4.32 - 4.39 (1H, m), 4.98 (1H, s), 6.87 (1H, dd), 7.03 (1H, td), 7.08 (1H, d), 7.09 - 7.12 (1H, m), 7.36 (1H, d), 7.41 - 7.46 (1H, m), 7.53 (1H, dd) |
| 21 | 4-[[4-(2,6-Dichlorophenoxy)-1-piperidinyl]methyl]-α-phenyl-1-piperidineacetic acid | 477/479 | δ_{(CD3OD)} 1.37 - 1.53 (2H, m), 1.67 - 1.80 (4H, m), 1.81 - 1.96 (3H, m), 2.19 (2H, d), 2.24 - 2.35 (2H, m), 2.56 - 2.74 (3H, m), 2.80 - 2.92 (2H, m), 3.61 - 3.80 (1H, m), 4.28 - 4.35 (1H, m), 4.38 - 4.46 (1H, m), 6.94 (1H, dd), 6.99 (1H, dd), 7.10 (1H, t), 7.31 - 7.37(3H, m), 7.43 - 7.50(2H, m) |
| 26 | (S)-4-[[4-(3,4-Dichlorophenoxy)-1-piperidinyl]methyl]-α-phenyl-piperidineacetic acid | 477/479 | δ_{(CD3OD + NaOD)} 1.25 - 1.38 (2H, m), 1.44 - 1.62 (2H, m), 1.68 - 2.06 (8H, m), 2.27 - 2.41 (4H, m), 2.69 - 2.78 (2H, m), 2.97 (1H, t), 4.35 - 4.47 (2H, m), 6.87 (1H, dd), 7.09 (1H, d), 7.37 (1H, d), 7.44 (3H, t), 7.54 - 7.59 (2H, m) |
| 28 | Methyl 4-[[4-(2,4-dichloro-3-methylphenoxy)-1-piperidinyl]methyl]-α-phenyl-1-piperidineacetate (α¹*R*)- | 505/507 | |
| 29 | (α¹*S*)-1,1-Dimethylethyl 4-[[4-(2,4-dichloro-3-methylphenoxy)-1-piperidinyl]methyl]-α-phenyl-1-piperidineacetate | 547/549 | |
| 30 | (α¹*R*)- Methyl 4-[[4-(3,4-dichloro-2-methylphenoxy)-1-piperidinyl]methyl]-α-phenyl-1-piperidineacetate | 505/507 | |
| 31 | (α¹*R*)- Methyl 4-[[4-(3,4-dichloro-2-methylphenoxy)-1-piperidinyl]methyl]-α-phenyl-1-piperidineacetate | 505/507 | |
| 32 | Methyl (*S*)-4-[[4-(3,4-dichlorophenoxy)-1-piperidinyl]methyl]-α-(1-methylethyl)-1-piperidineacetate | 457/459 | |
| 33 | 1,1-Dimethylethyl 4-[[4-(3,4-dichlorophenoxy)-1-piperidinyl]methyl]-α,α-dimethyl-1-piperidineacetate | 485/487 | |
| 35 | (α¹*R*)-4-[[4-(2,4-Dichloro-3-methylphenoxy)-1-piperidinyl]methyl]-α-phenyl-1-piperidineacetic acid | 491/493 | δ_{(CD3OD)} 1.43 - 1.63 (2H, m), 1.78 - 1.89 (4H, m), 1.91 - 2.05 (3H, m), 2.15 (1H, s), 2.32 (2H, d), 2.38 - 2.48 (5H, m), 2.69 - 2.80 (3H, m), 2.91 - 3.05 (2H, m), 4.40 - 4.52 (2H, m), 6.94 (1H, d), 7.25 (1H, d), 7.43 - 7.45 (3H, m), 7.55 - 7.58 (2H, m) |
| 36 | (α¹*S*)- 4-[[4-(2,4-Dichloro-3-methylphenoxy)-1-piperidinyl]methyl]-α-phenyl-1-piperidineacetic acid | 491/493 | δ_{(CD3OD)} 1.42 - 1.62 (2H, m), 1.77 - 1.90 (4H, m), 1.90 - 2.05 (4H, m), 2.30 (2H, d), 2.35 - 2.45 (5H, m), 2.72 (3H, t), 2.97 (2H, t), 4.40 - 4.49 (2H, m), 6.93 (1H, d), 7.25 (1H, d), 7.42 - 7.46 (3H, m), 7.53 - 7.59 (2H, m) |
| 37 | (α¹*R*)-4-[[4-(3,4-Dichloro-2-methylphenoxy)-1-piperidinyl]methyl]-α-phenyl-1-piperidineacetic acid | 491/493 | δ_{(CD3OD)} 1.43 - 1.67 (2H, m), 1.76 - 1.94 (4H, m), 1.95 - 2.10 (4H, m), 2.30 - 2.37 (5H, m), 2.45 (2H, t), 2.75 (3H, t), 3.01 (2H, t), 4.41 - 4.52 (2H, m), 6.93 (1H, d), 7.30 (1H, d), 7.47 (3H, dd), 7.59 (2H, q) |
| 38 | (α¹*S*)-4-[[4-(3,4-Dichloro-2-methylphenoxy)-1-piperidinyl]methyl]-α-phenyl-1-piperidineacetic acid | 491/493 | δ_{(CD3OD)} 1.30 - 1.54 (2H, m), 1.65 - 1.81 (4H, m), 1.81 - 1.98 (4H, m), 2.19 - 2.25 (5H, m), 2.28 - 2.39 (2H, m), 2.57 - 2.70 (3H, m), 2.82 - 2.98 (2H, m), 4.29 - 4.40 (2H, m), 6.81 (1H, d), 7.18 (1H, d), 7.32 - 7.39 (3H, m), 7.43 - 7.51 (2H, m) |
| 39 | (*S*)-4-[[4-(3,4-Dichlorophenoxy)-1-piperidinyl]methyl]-α-(1-methylethyl)-1-piperidineacetic acid | 443/445 | δ_{(CD3OD + NAOD)} 1.02 (3H, d), 1.14 (3H, d), 1.26 - 1.38 (3H, m), 1.42 - 1.64 (2H, m), 1.73 - 1.85 (2H, m), 1.98 - 2.07 (4H, m), 2.28 - 2.39 (2H, m), 2.40 - 2.49 (2H, m), 2.75 - 2.84 (2H, m), 2.97 - 3.04 (2H, m), 3.46 - 3.58 (2H, m), 4.39 - 4.46 (1H, m), 6.89 (1H, dd), 7.10 (1H, d), 7.38 (1H, d) |
| 40 | 4-[[4-(3,4-Dichlorophenoxy)-1-piperidinyl]methyl]-α,α-dimethyl-1-piperidineacetic acid | 429/431 | δ_{(CD3OD + NaOD)} 1.17 - 1.55 (8H, m), 1.61 - 1.77 (2H, m), 1.77 - 2.01 (7H, m), 2.23 - 2.40 (3H, m), 2.63 - 2.75 (2H, m), 2.88 (2H, t), 3.31 - 3.42 (1H, m), 4.28 - 4.39 (1H, m), 6.79 (1H, dd), 7.01 (1H, d), 7.28 (1H, d) |
| 43 | methyl 2-[(4-{[4-(2,4-Dichlorophenoxy)piperidin-1-yl]methyl}piperidin-1-yl)methyl]benzoate | 491/493 | |
| 51 | 2-[(4-{[4-(3,4-dichlorophenoxy)piperidin-1-yl]methyl}piperidin-1-yl)methyl]benzoic acid | 477/479 | δ_{(CD3OD)} 1.30 - 1.46 (2H, m), 1.70 - 1.83 (3H, m), 1.95 - 2.11 (4H, m), 2.25 - 2.41 (4H, m), 2.69 - 2.79 (2H, m), 2.94 (2H, t), 3.31 - 3.41 (2H, m), 4.26 (2H, s), 4.41 (1H, dt), 6.90 (1H, dd), 7.11 (1H, d), 7.39 (2H, d), 7.51 (2H, dtd), 7.97 (1H, dd) |
| 52 | 2-[[4-[[4-(2,4-Dichlorophenoxy)-1-piperidinyl]methyl]-1-piperidinyl]methyl]-benzoic acid | 477/479 | δ_{(CD3OD + NaOD)} 1.19 - 1.34 (2H, m), 1.47 - 1.61 (1H, m), 1.69 - 1.76 (2H, m), 1.76 - 1.86 (2H, m), 1.93 - 2.08 (4H, m), 2.22 (2H, d), 2.29 - 2.37 (2H, m), 2.64 - 2.72 (2H, m), 2.89 - 2.95 (2H, m), 3.83 (2H, s), 4.41 - 4.48 (1H, m), 7.07 (1H, d), 7.16 - 7.28 (3H, m), 7.38 (1H, d), 7.41 - 7.45 (2H, m) |
| 57 | 2-[[4-[[4-(3,4-Dichlorophenoxy)-1-piperidinyl]methyl]-1-piperidinyl]methyl]-5-fluoro-benzoic acid | 495/497 | δ _{(CD3OD)} 1.24 - 1.40 (2H, m), 1.66 - 1.77 (2H, m), 1.81 - 1.99 (5H, m), 2.34 (2H, d), 2.43 (2H, t), 2.71 - 2.79 (2H, m), 2.86 (2H, t), 3.22 - 3.29 (2H, m), 4.15 (2H, s), 4.31 - 4.39 (1H, m), 6.80 (1H, dd), 7.01 (1H, d), 7.11 (1H, td), 7.29 (1H, d), 7.31 (1H, dd), 7.56 (1H, dd) |
| 58 | 4-[[4-[[4-(2,4-Dichlorophenoxy)-1-piperidinyl]methyl]-1-piperidinyl]methyl]-3-fluoro-benzoic acid | 495/497 | δ_{(CD3OD)} 1.30 - 1.49 (2H, m), 1.82 - 1.96 (3H, m), 2.00 - 2.07 (2H, m), 2.11 - 2.22 (2H, m), 2.47 - 2.58 (2H, m), 2.69 (2H, d), 2.86 - 2.97 (2H, m), 3.03 - 3.22 (4H, m), 3.95 (2H, s), 4.60 - 4.69 (1H, m), 7.14 (1H, d), 7.28 (1H, dd), 7.44 - 7.51 (2H, m), 7.68 (1H, dd), 7.78 (1H, dd) |
| 59 | 2-[[4-[[4-(3,4-Dichlorophenoxy)-1-piperidinyl]methyl]-1-piperidinyl]methyl]-4-oxazolecarboxylic acid, | 468/470 | δ_{(CD3OD)} 1.20 - 1.32 (2H, m), 1.77 (3H, d), 1.92 - 2.00 (2H, m), 2.08 - 2.15 (4H, m), 2.81 (2H, d), 2.90 (2H, d), 3.11 - 3.22 (4H, m), 3.64 (2H, s), 4.55 (1H, s), 6.85 (1H, dd), 7.10 (1H, d), 7.31 (1H, d), 8.06 (1H, s) |
| 60 | 4-[[4-[[4-(3,4-Dichlorophenoxy)-1-piperidinyl]methyl]-1-piperidinyl]methyl]-benzoic acid | 477/479 | δ _{(CD3OD)} 1.29 (2H, q), 1.70 - 1.83 (5H, m), 1.93 - 2.01 (2H, m), 2.40 (2H, d), 2.44 - 2.58 (4H, m), 2.82 (2H, tt), 3.12 (2H, d), 3.88 (2H, s), 4.34 - 4.43 (1H, m), 6.81 (1H, dd), 7.03 (1H, d), 7.29 (1H, d), 7.33 (2H, d), 7.87 (2H, d) |
| 61 | 3-[[4-[[4-(3,4-Dichlorophenoxy)-1-piperidinyl]methyl]-1-piperidinyl]methyl]-benzoic acid | 477/479 | δ_{(CD3OD)} 1.25 - 1.41 (2H, m), 1.66 - 1.78 (3H, m), 1.82 - 1.99 (4H, m), 2.32 (2H, d), 2.37 - 2.47 (2H, m), 2.59 - 2.79 (4H, m), 3.25 (2H, s), 4.00 (2H, s), 4.30 - 4.39 (1H, m), 6.79 (1H, dd), 7.01 (1H, d), 7.28 (1H, d), 7.31 - 7.42 (2H, m), 7.87 - 7.92 (2H, m) |
| 62 | 2-[[4-[[4-(2,4-Dichloro-3-methylphenoxy)-1-piperidinyl]methyl]-1-piperidinyl]methyl]-benzoic acid | 491/493 | δ_{(CD3OD)} 1.31 1.50 (2H, m), 1.81 - 1.93 (3H, m), 1.96 - 2.11 (4H, m), 2.39 (2H, d), 2.45 - 2.55 (5H, m), 2.77 - 2.85 (2H, m), 2.90 - 3.03 (2H, m), 3.34 - 3.40 (2H, m), 4.27 (2H, s), 4.46 - 4.54 (1H, m), 6.97 (1H, d), 7.28 (1H, d), 7.38-7.41 (1H, m), 7.51 (2H, dtd), 7.98 (1H, dd) |
| 63 | 2-[[4-[[4-(3,4-Dichloro-2-methylphenoxy)-1-piperidinyl]methyl]-1-piperidinyl]methyl]-benzoic acid | 491/493 | δ_{(CD3OD)} 1.26 - 1.42 (2H, m), 1.74 - 1.85 (3H, m), 1.92 - 2.09 (4H, m), 2.24 - 2.32 (2H, m), 2.30 (3H, s), 2.38 (2H, t), 2.69 (2H, t), 2.91 (2H, t), 3.30 - 3.39 (2H, m), 4.19 - 4.26 (2H, m), 4.37 - 4.47 (1H, m), 6.90 (1H, d), 7.26 (1H, d), 7.36 (1H, d), 7.48 (2H, quintetd), 7.95 (1H, d) |
| 64 | [2-[[4-[[4-(2,4-Dichloro-3-methylphenoxy)-1-piperidinyl]methyl]-1-piperidinyl]methyl]phenoxy]-acetic acid | 521/523 | δ_{(CD3OD)} 1.46 - 1.63 (2H, m), 1.69 - 1.95 (7H, m), 2.23 - 2.29 (2H, m), 2.31 - 2.40 (5H, m), 2.63 - 2.73 (2H, m), 2.79 - 2.91 (2H, m), 3.35 (2H, d), 4.11 (2H, s), 4.34 - 4.42 (1H, m), 4.57 (2H, s), 6.85 (1H, d), 6.94 (1H, t), 7.07 (1H, d), 7.16 (1H, d), 7.24 (1H, d), 7.35 (1H, t) |
| 65 | 2-[[4-[[4-(2,4-Dichlorophenoxy)-1-piperidinyl]methyl]-1-piperidinyl]methyl]-benzenesulfonic acid | (ES -ve) 511/513 | δ_{(CD3OD)} 1.18 - 1.40 (2H, m), 1.69 - 1.82 (3H, m), 1.89 - 2.02 (4H, m), 2.25 (2H, d), 2.31 - 2.41 (2H, m), 2.63 - 2.73 (2H, m), 2.98 (2H, t), 3.34 (2H, d), 4.34 - 4.44 (1H, m), 4.52 (2H, s), 6.98 (1H, d), 7.14 (1H, dd), 7.30 (1H, d), 7.39 - 7.51 (3H, m), 7.91 - 7.96 (1H, m) |
| 66 | 2-[[4-[[4-(2,4-Dichloro-3-methylphenoxy)-1-piperidinyl]methyl]-1-piperidinyl]methyl]-benzenesulfonic acid | 527/529 | δ_{(CD3OD)} 1.15 - 1.33 (2H, m), 1.66 - 2.02 (7H, m), 2.12 - 2.37 (7H, m), 2.57 - 2.68 (2H, m), 2.88 - 3.02 (2H, m), 3.28 - 3.40 (2H, m), 4.31 - 4.40 (1H, m), 4.51 (2H, s), 6.84 (1H, d), 7.15 (1H, d), 7.39 - 7.50 (3H, m), 7.90 - 7.96 (1H, m) |
| 68 | Methyl4-[[4-(2,4-dichloro-3-methylphenoxy)-1-piperidinyl]methyl]-1-piperidineacetate | 429/431 | |
| 69 | Methyl-4-[[4-(4-chloro-2-methylphenoxy)-1-piperidinyl]methyl]-1-piperidineacetate | (ES-ve) 393/395 | |
| 70 | Methyl 4-[[4-(2,4-dichlorophenoxy)-1-piperidinyl]methyl]-1-piperidineacetate | 415/417 | |
| 71 | Methyl 4-[[4-(3,4-dichloro-2-methylphenoxy)-1-piperidinyl]methyl]-1-piperidineacetate | 429/433 | |
| 72 | Methyl 4-[[4-(3,4-dichlorophenoxy)-1-piperidinyl]methyl]-4-hydroxy-1-piperidineacetate | 431/433 | |
| 74 | Methyl (2*S*)-2-(4-{[4-(3,4-dichlorophenoxy)piperidin-1-yl]methyl}piperidin-1-yl)propanoate | 429/431 | |
| 75 | (αR)-Methyl-4-[[4-(4-chloro-2-methylphenoxy)-1-piperidinyl]methyl]-α-methyl-1-piperidineacetate | (ES-ve) 407/409 | |
| 76 | (α*S*)-Methyl-4-[[4-(4-chloro-2-methylphenoxy)-1-piperidinyl]methyl]-α-methyl-1-piperidineacetate | (ES-ve) 407/409 | |
| 78 | 4-[[4-(2,4-Dichloro-3-methylphenoxy)-1-piperidinyl]methyl]-1-piperidineacetic acid | 415/417 | δ_{(CDCl3)} 1.45 - 1.61 (2H, m), 1.80 - 1.93 (3H, m), 1.96 - 2.10 (4H, m), 2.33 (2H, d), 2.38 - 2.48 (2H, m), 2.47 (3H, s), 2.71 - 2.82 (2H, m), 3.01 (2H, t), 3.55 - 3.68 (2H, m), 3.59 (2H, s), 4.44 - 4.54 (1H, m), 6.96 (1H, d), 7.27 (1H, d) |
| 79 | 4-[[4-(4-Chloro-2-methylphenoxy)-1-piperidinyl]methyl]-1-piperidineacetic acid | | δ_{(CD3OD)} 1.45 - 1.61 (2H, m), 1.76 - 1.94 (3H, m), 1.97 - 2.09 (4H, m), 2.20 (3H, s), 2.32 (2H, d), 2.40 (2H, t), 2.69 - 2.78 (2H, m), 3.01 (2H, t), 3.57 - 3.66 (4H, m), 4.37 - 4.46 (1H, m), 6.89 (1H, d), 7.08 - 7.14 (2H, m) |
| 80 | [[4-(2,4-Dichlorophenoxy)-1-piperidinyl]methyl)-1-piperidineacetic acid | | δ_{(CD3OD)} 1.32 - 1.48 (2H, m), 1.67 - 1.80 (3H, m), 1.84 - 1.96 (4H, m), 2.20 (2H, d), 2.25 - 2.34 (2H, m), 2.59 - 2.68 (2H, m), 2.83 - 2.95 (2H, m), 3.45 - 3.55 (4H, m), 4.34 - 4.42 (1H, m), 6.98 (1H, d), 7.14 (1H, dd), 7.30 (1H, d) |
| 81 | 4-[[4-(3,4-Dichloro-2-methylphenoxy)-1-piperidinyl]methyl]-1-piperidineacetic acid | 415/417 | δ_{(CD3OD)} 1.52 (2H, dd), 1.79 - 1.94 (3H, m), 1.99 - 2.08 (4H, m), 2.32 (3H, s), 2.38 (2H, d), 2.48 (2H, t), 2.77 (2H, t), 3.01 (2H, t), 3.55 - 3.64 (4H, m), 4.41 - 4.50 (1H, m), 6.93 (1H, d), 7.28 (1H, d) |
| 82 | 4-[[4-(3,4-Dichlorophenoxy)-1-piperidinyl]methyl]-4-hydroxy-1-piperidineacetic acid | 417/419 | |
| 83 | (2*R*)-2-(4-{[4-(3,4-Dichlorophenoxy)piperidin-1-yl]methyl}piperidin-1-yl)propanoic acid | 415/417 | δ_{(CD3OD)} 1.42 - 1.59 (5H, m), 1.71 - 2.12 (7H, m), 2.28 - 2.41 (4H, m), 2.70 - 2.80 (2H, m), 2.93 - 3.14 (2H, m), 3.49 - 3.62 (3H, m), 4.37 - 4.46 (1H, m), 6.91 (1H, dd), 7.12 (1H, d), 7.40 (1H, t) |
| 84 | (2*S*)-2-(4-{[4-(3,4-Dichlorophenoxy)piperidin-1-yl]methyl}piperidin-1-yl)propanoic acid | 415/417 | δ _{(CD3OD)} 1.44 - 1.60 (5H, m), 1.73 - 2.12 (7H, m), 2.30 - 2.43 (4H, m), 2.71 - 2.81 (2H, m), 2.93 - 3.14 (2H, m), 3.50 - 3.62 (3H, m), 4.38 - 4.48 (1H, m), 6.91 (1H, dd), 7.12 (1H, d), 7.40 (1H, d) |
| 85 | (α*R*)- 4-[[4-(4-Chloro-2-methylphenoxy)-1-piperidinyl]methyl]-α-methyl-1-piperidineacetic acid | | δ_{(CD3OD)} 1.45 - 1.61 (5H, m), 1.76 - 2.13 (7H, m), 2.20 (3H, s), 2.32 (2H, d), 2.41 (2H, t), 2.69 - 2.79 (2H, m), 2.94 - 3.14 (2H, m), 3.50 - 3.62 (3H, m), 4.37 - 4.46 (1H, m), 6.90 (1H, d), 7.08 - 7.14 (2H, m) |
| 86 | (αS)-4-[[4-(4-Chloro-2-methylphenoxy)-1-piperidinyl]methyl]-α-methyl-1-piperidineacetic acid | | δ_{(CD3OD)} 1.45 - 1.55 (5H, m), 1.75 - 1.91 (3H, m), 1.95 - 2.09 (4H, m), 2.18 (3H, s), 2.28 (2H, d), 2.37 (2H, t), 2.67 - 2.74 (2H, m), 2.92 - 3.09 (2H, m), 3.49 - 3.58 (3H, m), 4.35 - 4.42 (1H, m), 6.87 (1H, d), 7.06 - 7.12 (2H, m) |
| 88 | Methyl-4-[[4-(4-chloro-2-methylphenoxy)-1-piperidinyl]methyl]-α,α-dimethyl-1-piperidinepropanoate | 437/439 | |
| 89 | Methyl 4-[[4-(2,4-dichloro-3-methylphenoxy)-1-piperidinyl]methyl]-α,α-dimethyl-1-piperidinepropanoate | 471/473 | |
| 91 | 4-[[4-(4-Chloro-2-methylphenoxy)-1-piperidinyl]methyl]-α,α-dimethyl-1-piperidinepropanoic acid | 423/425 | δ(_{CD3OD}) 1.14 (6H, s), 1.32 - 1.47 (2H, m), 1.65 - 1.80 (3H, m), 1.92 (4H, d), 2.08 (3H, s), 2.22 (2H, d), 2.27 - 2.36 (2H, m), 2.59 - 2.68 (2H, m), 2.86 - 2.99 (4H, m), 3.37 - 3.45 (2H, m), 4.26 - 4.34 (1H, m), 6.78 (1H, d), 6.97 (1H, d), 7.01 (1H, q) |
| 92 | 4-[[4-(2,4-Dichloro-3-methylphenoxy)-1-piperidinyl]methyl]-α,α-dimethyl-1-piperidinepropanoic acid | 457/459 | δ_{(CD3OD + NaOD)} 1.10 (6H, s), 1.21 - 1.36 (4H, m), 1.59 - 1.70 (2H, m), 1.77 - 1.87 (2H, m), 1.93 - 2.02 (2H, m), 2.04 - 2.13 (2H, m), 2.18 - 2.23 (2H, m), 2.29 - 2.38 (1H, m), 2.44 (3H, s), 2.48 (2H, s), 2.64 - 2.73 (2H, m), 2.87 - 2.93 (2H, m), 4.41 - 4.48 (1H, m), 6.94 (1H, d), 7.24 (1H, d) |
| 95 | Methyl 4-[[4-(3,4-dichlorophenoxy)-1-piperidinyl]methyl]- 1-piperidinepentanoate | 457/459 | |
| 96 | Ethyl 4-[[4-(3,4-dichlorophenoxy)-1-piperidinyl]methyl]-1-piperidinehexanoate | 485/487 | |
| 97 | 4-[[4-(3,4-Dichlorophenoxy)-1-piperidinyl]methyl]-1-piperidinebutanoic acid | 429/431 | δ_{(CD3OD + NaOD)} 1.08 - 1.26 (2H, m), 1.42 - 1.53 (1H, m), 1.61 - 1.77 (6H, m), 1.85 - 1.97 (4H, m), 2.05 (2H, t), 2.13 (2H, d), 2.17 - 2.24 (2H, m), 2.24 - 2.32 (2H, m), 2.56 - 2.66 (2H, m), 2.87 (2H, d), 4.24 - 4.32 (1H, m), 6.78 (1H, dd), 6.99 (1H, d), 7.27 (1H, d) |
| 98 | 4-[[4-(3,4-Dichlorophenoxy)-1-piperidinyl]methyl]- 1-piperidinepentanoic acid | 443/445 | δ_{(CD3OD + NaOD)} 1.15-1.29 (1H, m), 1.36 (2H, q), 1.55 - 1.62 (2H, m), 1.62 - 1.72 (4H, m), 1.86 - 1.98 (4H, m), 2.16 (2H, t), 2.22 (2H, d), 2.28 (2H, t), 2.61 - 2.70 (2H, m), 2.78 (2H, t), 2.93 (2H, t), 3.36 - 3.44 (2H, m), 4.27 - 4.34 (1H, m), 6.79 (1H, dd), 7.00 (1H, d), 7.28 (1H, d) |
| 99 | 4-[[4-(3,4-Dichlorophenoxy)-1-piperidinyl]methyl]- 1-piperidinehexanoic acid | 457/459 | δ_{(CD3OD + NaOD}) 1.22 - 1.42 (4H, m), 1.49 - 1.71 (5H, m), 1.74 - 1.85 (4H, m), 1.94 - 2.07 (4H, m), 2.14 - 2.27 (4H, m), 2.29 - 2.40 (4H, m), 2.67 - 2.77 (2H, m), 2.93 - 3.01 (2H, m), 4.35 - 4.44 (1H, m), 6.90 (1H, dd), 7.10 (1H, d), 7.39 (1H, d) |
| 101 | 4-(3,4-Dichlorophenoxy)-1-[[1-[2-(2H-tetrazol-5-yl)ethyl]-4-piperidinyl]methyl]-piperidine | 439/441 | δ_{(CD3OD)} 1.35 (2H, dd), 1.66 - 1.76 (2H, m), 1.77 - 1.84 (1H, m), 1.88 - 1.99 (4H, m), 2.31 (2H, d), 2.34 - 2.46 (2H, m), 2.66 - 2.89 (4H, m), 3.12 - 3.18 (2H, m), 3.24 - 3.36 (2H, m), 3.45 (2H, d), 4.19 - 4.43 (1H, m), 6.80 (1H, dd), 7.02 (1H, d), 7.29 (1H, d) |
| 103 | *N*-[3-[4-[[4-(3,4-Dichlorophenoxy)-1-piperidinyl]methyl]-1-piperidinyl]propyl]-1,1,1-trifluoro-methanesulfonamide | 532/534 | δ_{(CD3OD)} 1.14 - 1.30 (2H, m), 1.53 - 1.76 (5H, m), 1.80 (2H, d), 1.86 - 1.97 (2H, m), 2.18 (2H, d), 2.21 - 2.34 (4H, m), 2.64 (4H, t), 3.10 (2H, d), 3.17 (2H, t), 4.20 - 4.40 (1H, m), 6.79 (1H, dd), 7.00 (1H, d), 7.28 (1H, d) |
| 104 | *N*-[2-[4-[[4-(3,4-Dichloro-2-methylphenoxy)-1-piperidinyl]methyl]-1-piperidinyl]ethyl]-1,1,1-trifluoro-methanesulfonamide, | 532/534 | δ_{(CD3OD)} 1.27 - 1.43 (2H, m), 1.69 - 1.81 (1H, m), 1.82 - 1.92 (4H, m), 1.99 - 2.10 (2H, m), 2.32 (3H, s), 2.39 - 2.48 (4H, m), 2.51 - 2.62 (2H, m), 2.77 (2H, t), 2.80 - 2.89 (2H, m), 3:19 (2H, d), 3.36 (2H, t), 4.43 - 4.53 (1H, m), 6.92 (1H, d), 7.28 (1H, d) |

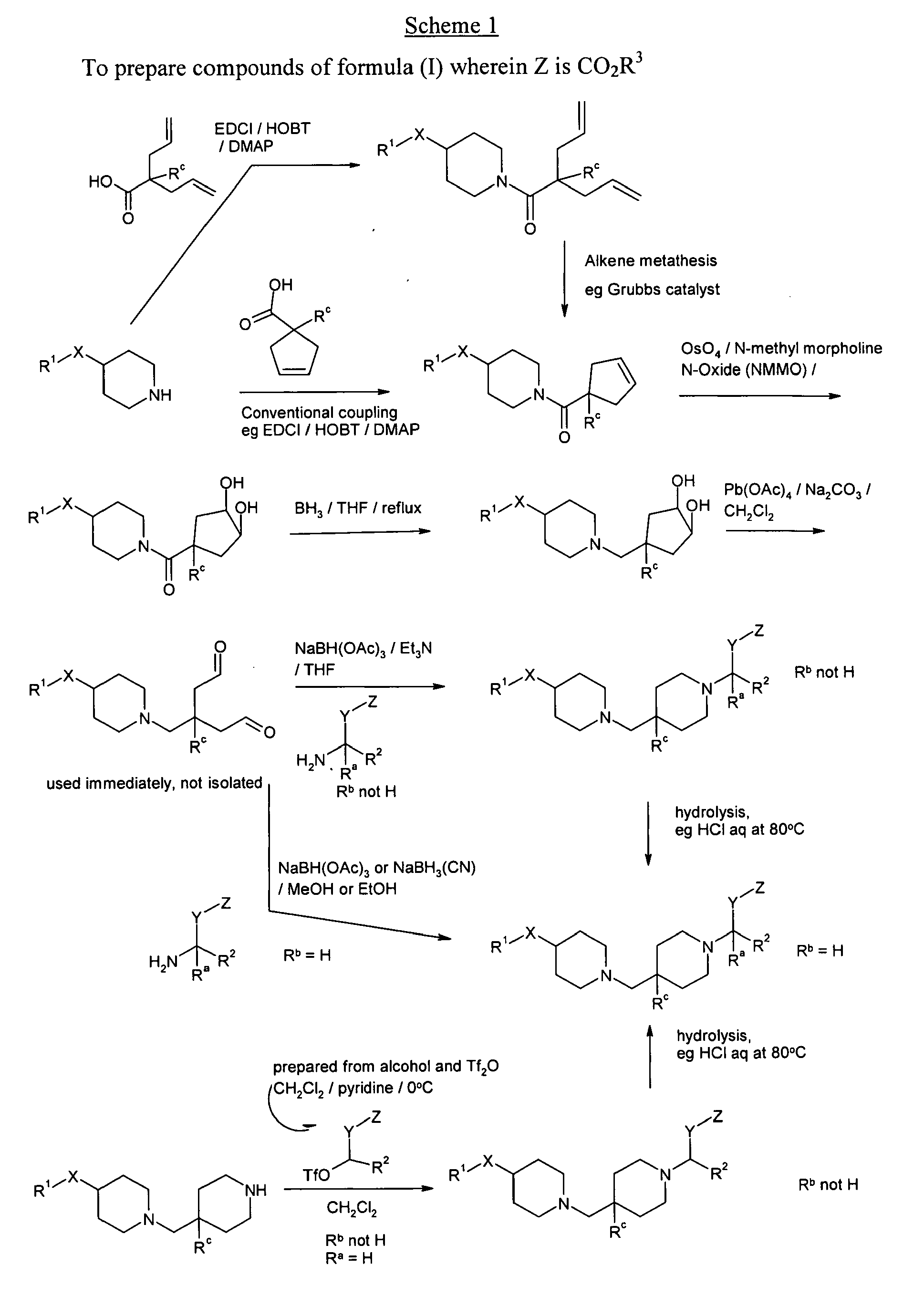

## Claims

1. A compound of formula (A), (B) or (C): 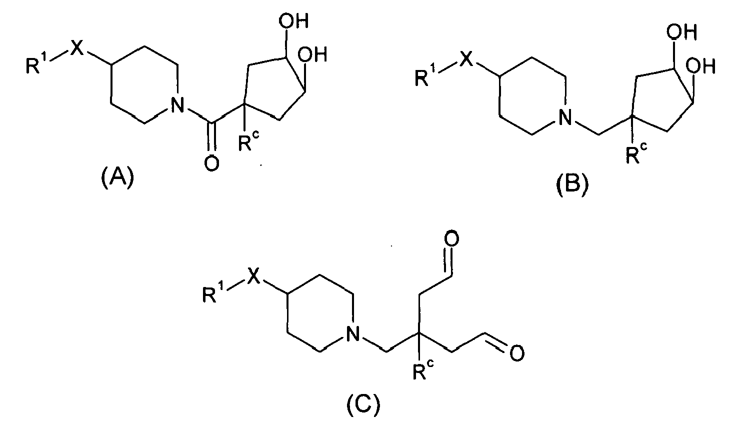 wherein:
X is CH₂, C(O), O, S, S(O), S(O)₂ or NR³;
R¹ is hydrogen, C₁₋₆ alkyl, aryl or heterocyclyl;
R^{c} is hydrogen or hydroxy;
wherein, unless stated otherwise, the foregoing aryl and heterocyclyl moieties are optionally substituted by: halogen, cyano, nitro, hydroxy, oxo, S(O)ₚR⁴, OC(O)NR⁵R⁶, NR⁷R⁸, NR⁹C(O)R¹⁰, NR¹¹C(O)NR¹²R¹³, S(O)₂NR¹⁴R¹⁵, NR¹⁶S(O)₂R¹⁷, C(O)NR¹⁸R¹⁹, C(O)R²⁰, CO₂R²¹, NR²²CO₂R²³, C₁₋₆ alkyl, CF₃, C₁₋₆ alkoxy(C₁₋₆)alkyl, C₁₋₆ alkoxy, OCF₃, C₁₋₆ alkoxy(C₁₋₆)alkoxy, C₁₋₆ alkylthio, C₂₋₆ alkenyl, C₂₋₆ alkynyl, C₃₋₁₀ cycloalkyl (itself optionally substituted by C₁₋₄ alkyl or oxo), methylenedioxy, difluoromethylenedioxy, phenyl, phenyl(C₁₋₄)alkyl, phenoxy, phenylthio, phenyl(C₁₋₄)alkoxy, heterocyclyl, heterocyclyl(C₁₋₄)alkyl, heterocyclyloxy or heterocyclyl(C₁₋₄)alkoxy; wherein any of the immediately foregoing phenyl and heterocyclyl moieties are optionally substituted with halogen, hydroxy, nitro, S(O)_{q}(C₁₋₄ alkyl), S(O)₂NH₂, S(O)₂NH(C₁₋₄ alkyl), S(O)₂N(C₁₋₄ alkyl)₂ (and these alkyl groups may join to form a ring as described for R⁵ and R⁶ below), cyano, C₁₋₄ alkyl, C₁₋₄ alkoxy, C(O)NH₂, C(O)NH(C₁₋₄ alkyl), C(O)N(C₁₋₄ alkyl)₂ (and these alkyl groups may join to form a ring as described for R⁵ and R⁶ below), CO₂H, CO₂(C₁₋₄ alkyl), NHC(O)(C₁₋₄ alkyl), NES(O)₂(C₁₋₄ alkyl), C(O)(C₁₋₄ alkyl), CF₃ or OCF₃;
p and q are, independently, 0, 1 or 2;
R³, R⁵, R⁶, R⁷, R⁸, R⁹, R¹⁰, R¹¹, R¹², R¹³, R¹⁴, R¹⁵, R¹⁶, R¹⁸, R¹⁹, R²⁰, R²¹ and R²² are, independently, hydrogen, C₁₋₆ alkyl (optionally substituted by halogen, hydroxy or C₃₋₁₀ cycloalkyl), CH₂(C₂₋₆ alkenyl), phenyl (itself optionally substituted by halogen, hydroxy, nitro, NH₂, NH(C₁₋₄ alkyl), N(C₁₋₄ alkyl)₂ (and these alkyl groups may join to form a ring as described for R⁵ and R⁶ below), S(O)₂(C₁₋₄ alkyl), S(O)₂NH₂, S(O)₂NH(C₁₋₄ alkyl), S(O)₂N(C₁₋₄ alkyl)₂ (and these alkyl groups may join to form a ring as described for R⁵ and R⁶ below), cyano, C₁₋₄ alkyl, C₁₋₄ alkoxy, C(O)NH₂, C(O)NH(C₁₋₄ alkyl), C(O)N(C₁₋₄ alkyl)₂(and these alkyl groups may join to form a ring as described for R⁵ and R⁶ below), CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃) or heterocyclyl (itself optionally substituted by halogen, hydroxy, nitro, NH₂, NH(C₁₋₄ alkyl), N(C₁₋₄ alkyl)₂ (and these alkyl groups may join to form a ring as described for R⁵ and R⁶ below), S(O)₂(C₁₋₄ alkyl), S(O)₂NH₂, S(O)₂NH(C₁₋₄ alkyl), S(O)₂N(C₁₋₄ alkyl)₂ (and these alkyl groups may join to form a ring as described for R⁵ and R⁶ below), cyano, C₁₋₄ alkyl, C₁₋₄ alkoxy, C(O)NH₂, C(O)NH(C₁₋₄ alkyl), C(O)N(C₁₋₄ alkyl)₂ (and these alkyl groups may join to form a ring as described for R⁵ and R⁶ below), CO₂H CO₂(C₁₋₄ alkyl), NHC(O)(C₁₋₄ alkyl), NES(O)₂(C₁₋₄ alkyl), C(O)(C₁₋₄ alkyl), CF₃ or OCF₃);
alternatively NR⁵R⁶, NR⁷R⁸, NR¹²R¹³, NR¹⁴R¹⁵, NR¹⁸R¹⁹, may, independently, form a 4-7 membered heterocyclic ring, azetidine, pyrrolidine, piperidine, azepine, morpholine or piperazine, the latter optionally substituted by C₁₋₄ alkyl on the distal nitrogen;
R⁴, R¹⁷ and R²³ are, independently, C₁₋₆ alkyl (optionally substituted by halogen, hydroxy or C₃₋₁₀ cycloalkyl), CH₂(C₂₋₆ alkenyl), phenyl (itself optionally substituted by halogen, hydroxy, nitro, NH₂, NH(C₁₋₄ alkyl), N(C₁₋₄ alkyl)₂ (and these alkyl groups may join to form a ring as described for R⁵ and R⁶ above), S(O)₂(C₁₋₄ alkyl), S(O)₂NH₂, S(O)₂NH(C₁₋₄ alkyl), S(O)₂N(C₁₋₄ alkyl)₂ (and these alkyl groups may join to form a ring as described for R⁵ and R⁶ above), cyano, C₁₋₄ alkyl, C₁₋₄ alkoxy, C(O)NH₂, C(O)NH(C₁₋₄ alkyl), C(O)N(C₁₋₄ alkyl)₂ (and these alkyl groups may join to form a ring as described for R⁵ and R⁶ above), CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃) or heterocyclyl (itself optionally substituted by halogen, hydroxy, nitro, NH₂, NH(C₁₋₄ alkyl), N(C₁₋₄ alkyl)₂ (and these alkyl groups may join to form a ring as described for R⁵ and R⁶ above), S(O)₂(C₁₋₄ alkyl), S(O)₂NH₂, S(O)₂NH(C₁₋₄ alkyl), S(O)₂N(C₁₋₄ alkyl)₂ (and these alkyl groups may join to form a ring as described for R⁵ and R⁶ above), cyano, C₁₋₄ alkyl, C₁₋₄ alkoxy, C(O)NH₂, C(O)NH(C₁₋₄ alkyl), C(O)N(C₁₋₄ alkyl)₂ (and these alkyl groups may join to form a ring as described for R⁵ and R⁶ above), CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃).

2. A compound as claimed in claim 1 wherein R¹ is phenyl optionally substituted with halogen, C₁₋₄ alkyl or C₁₋₄ alkoxy.

3. A compound as claimed in claim 1 or 2 wherein X is O.

4. A compound as claimed in claim 1, 2 or 3 wherein R^{c} is hydrogen.

5. A process for preparing a compound of formula (A) as claimed in claim 1, the process comprising reacting a compound of formula (D): 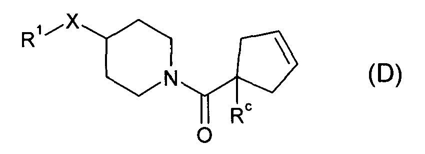 with osmium tetra-oxide in the presence of N-methyl-morpholine N-oxide.

6. A process for preparing a compound of formula (B) as claimed in claim 1, the process comprising reacting a compound of formula (A) with boron trihydride in tetrahydrofuran at reflux.

7. A process for preparing a compound of formula (C) as claimed in claim 1, the process comprising reacting a compound of formula (B) with lead tetra-acetate in the presence of sodium carbonate and using dichloromethane as solvent.
